(19) Europäisches Patentamt
European Patent Office
Office européen des brevets

(11) EP 1 691 814 B1

(12) **EUROPEAN PATENT SPECIFICATION**

(45) Date of publication and mention of the grant of the patent:
**25.07.2012 Bulletin 2012/30**

(21) Application number: **04798708.6**

(22) Date of filing: **30.11.2004**

(51) Int Cl.:
***A61K 31/55*** (2006.01) ***C07D 223/10*** (2006.01)
***C07D 223/12*** (2006.01) ***C07K 5/06*** (2006.01)
***C07K 5/08*** (2006.01) ***A61P 29/00*** (2006.01)
***A61P 25/28*** (2006.01) ***A61P 37/00*** (2006.01)
***A61P 19/00*** (2006.01)

(86) International application number:
**PCT/GB2004/005030**

(87) International publication number:
**WO 2005/053702 (16.06.2005 Gazette 2005/24)**

(54) **ANTI-INFLAMMATORY AGENTS**

ENTZÜNDUNGSHEMMENDE MITTEL

AGENTS ANTI-INFLAMMATOIRES

(84) Designated Contracting States:
**AT BE BG CH CY CZ DE DK EE ES FI FR GB GR HU IE IS IT LI LU MC NL PL PT RO SE SI SK TR**

(30) Priority: **01.12.2003 GB 0327775**
**05.08.2004 GB 0417436**
**10.08.2004 GB 0417734**

(43) Date of publication of application:
**23.08.2006 Bulletin 2006/34**

(60) Divisional application:
**10011516.1 / 2 279 741**

(73) Proprietor: **Cambridge Enterprise Limited**
**Trinity Lane**
**Cambridge**
**Cambridgeshire CB2 1TN (GB)**

(72) Inventors:
- **GRAINGER, David, John,**
**Department of Medicine**
**Cambridge CB2 2QQ (GB)**
- **FOX, David, John,**
**Department of Chemistry**
**Cambridge, CB2 1EW (GB)**

(74) Representative: **Roberts, Michael Austin**
**Reddie & Grose**
**16 Theobalds Road**
**London**
**WC1X 8PL (GB)**

(56) References cited:
**EP-A- 0 351 856**
**EP-A- 0 351 856**
**EP-A- 0 557 936**
**EP-A- 1 008 346**
**EP-A- 1 008 346**
**EP-A1- 0 297 610**
**EP-A2- 0 312 157**
**WO-A-00/02903**
**WO-A-01/68655**
**WO-A-01/68655**
**WO-A-97/16410**
**WO-A-97/21690**
**WO-A-98/07694**
**WO-A-98/07694**
**WO-A-98/17680**
**WO-A-98/17680**
**WO-A1-00/07995**
**JP-A- 53 121 781**
**JP-A- 2001 002 649**
**US-A- 4 284 646**
**US-A- 5 362 718**
**US-A- 5 821 231**
**US-A- 5 821 231**
**US-A- 5 852 007**
**US-A- 5 852 007**
**US-B1- 6 344 450**
**US-B1- 6 344 450**

- **DAVIDSON BRADLEY S ET AL: "Isolation and synthesis of caprolactins A and B, new caprolactams from a marine bacterium" TETRAHEDRON, vol. 49, no. 30, 1993, pages 6569-6574, XP002320426 ISSN: 0040-4020**
- **SEIFFERT D ET AL: "Presenilin-1 and -2 are molecular targets for gamma-secretase inhibitors." THE JOURNAL OF BIOLOGICAL CHEMISTRY. 3 NOV 2000, vol. 275, no. 44, 3 November 2000 (2000-11-03), pages 34086-34091, XP002320427 ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE CAPLUS [Online] CHEMICAL ABSTRACTS SERVICE, COLUMBUS, OHIO, US; 1987, FUJINO, TOSHIHIRO; NAKAI, AKIRA; MATSUKUMA, YOSHIHISA: ".alpha.-N-Acyllysine derivatives" XP002413883 Database accession no. 1987:407608 & JP 62 005994 A (TORAY INDUSTRIES) 12 January 1987 (1987-01-12)
- DATABASE CA [Online] CHEMICAL ABSTRACTS SERVICE, COLUMBUS, OHIO, US; KIMURA, KOICHI ET AL: "Anodic synthesis of fatty acids. II. The syntheses of 3,3-dimethylated branched acids" XP002413884 retrieved from STN Database accession no. 1959:39476 & CHEMICAL & PHARMACEUTICAL BULLETIN, CODEN: CPBTAL; ISSN: 0009-2363, vol. 6, 1958, pages 9-12,
- DATABASE CA [Online] CHEMICAL ABSTRACTS SERVICE, COLUMBUS, OHIO, US; D'IPPOLITO, GIULIANA ET AL: "Detection of short-chain aldehydes in marine organisms: the diatom Thalassiosira rotula" XP002413885 retrieved from STN Database accession no. 2002:586183 & TETRAHEDRON LETTERS, CODEN: TELEAY; ISSN: 0040-4039, vol. 43, no. 35, 2002, pages 6137-6140,
- DATABASE CA [Online] CHEMICAL ABSTRACTS SERVICE, COLUMBUS, OHIO, US; AIDA, YOKO ET AL: "The antagonistic effects of khellactones on platelet-activating factor, histamine, and leukotriene D4" XP002413886 retrieved from STN Database accession no. 1995:741644 & CHEMICAL & PHARMACEUTICAL BULLETIN, CODEN: CPBTAL; ISSN: 0009-2363, vol. 43, no. 5, 1995, pages 859-867,
- DATABASE CA [Online] CHEMICAL ABSTRACTS SERVICE, COLUMBUS, OHIO, US; SASMAL, SANJITA ET AL: "Synthesis of Cyclic Peptidomimetics from Aldol Building Blocks" XP002413887 retrieved from STN Database accession no. 2002:548516 & JOURNAL OF ORGANIC CHEMISTRY, CODEN: JOCEAH; ISSN: 0022-3263, vol. 67, no. 17, 2002, pages 6260-6263,
- DATABASE CA [Online] CHEMICAL ABSTRACTS SERVICE, COLUMBUS, OHIO, US; HERMANN, CHRISTOPH ET AL: "Syntheses of hapalosin analogs by solid-phase assembly of acyclic precursors" XP002413888 retrieved from STN Database accession no. 2001:749941 & TETRAHEDRON ( 2001 ), CODEN: TETRAB; ISSN: 0040-4020, vol. 57, no. 43, 2001, pages 8999-9010,
- DATABASE CA [Online] CHEMICAL ABSTRACTS SERVICE, COLUMBUS, OHIO, US; LI, WEI-DONG Z. ET AL: "Chemoselective Aldol Reaction of Silyl Enolates Catalyzed by MgI2 Etherate" XP002413889 retrieved from STN Database accession no. 2002:667580 & ORGANIC LETTERS ( 2002 ), CODEN: ORLEF7; ISSN: 1523-7060, vol. 4, no. 20, 2002, pages 3485-3488,
- DATABASE CA [Online] CHEMICAL ABSTRACTS SERVICE, COLUMBUS, OHIO, US; HOMMA, KOICHI ET AL: "Stereoselective syntheses of .alpha.-substituted cyclic ethers and syn-1,3-diols" XP002413890 retrieved from STN Database accession no. 1991:6220 & BULLETIN OF THE CHEMICAL SOCIETY OF JAPAN, CODEN: BCSJA8; ISSN: 0009-2673, vol. 63, no. 7, 1990, pages 1898-1915,
- DAVIDSON BRADLEY S ET AL: "Isolation and synthesis of caprolactins A and B, new caprolactams from a marine bacterium" TETRAHEDRON, vol. 49, no. 30, 1993, pages 6569-6574, XP002320426 ISSN: 0040-4020
- SEIFFERT D ET AL: "Presenilin-1 and -2 are molecular targets for gamma-secretase inhibitors." THE JOURNAL OF BIOLOGICAL CHEMISTRY. 3 NOV 2000, vol. 275, no. 44, 3 November 2000 (2000-11-03), pages 34086-34091, XP002320427 ISSN: 0021-9258
- JAKUBKE H-D: "PEPTIDE CHEMIE UND BIOLOGIE, 4.7.8. PEPTIDMIMETICA UND ANDERE PSEUDOPEPTIDE", PEPTIDE CHEMIE UND BIOLOGIE, XX, XX, 1 January 1996 (1996-01-01), pages 286-296, XP001543007,

## Description

The invention relates to the use of 3-aminocaprolactam derivatives for preparing a medicament intended to prevent or treat inflammatory disorders.

Inflammation is an important component of physiological host defence. Increasingly, however, it is clear that temporally or spatially inappropriate inflammatory responses play a part in a wide range of diseases, including those with an obvious leukocyte component (such as autoimmune diseases, asthma or atherosclerosis) but also in diseases that have not traditionally been considered to involve leukocytes (such as osteoporosis or Alzheimer's disease).

The chemokines are a large family of signalling molecules with homology to interleukin-8 which have been implicated in regulating leukocyte trafficking both in physiological and pathological conditions. With more than fifty ligands and twenty receptors involved in chemokine signalling, the system has the requisite information density to address leukocytes through the complex immune regulatory processes from the bone marrow, to the periphery, then back through secondary lymphoid organs. However, this complexity of the chemokine system has at first hindered pharmacological approaches to modulating inflammatory responses through chemokine receptor blockade. It has proved difficult to determine which chemokine receptor(s) should be inhibited to produce therapeutic benefit in a given inflammatory disease.

More recently, a family of agents which block signalling by a wide range of chemokines simultaneously has been described: Reckless et al., Biochem J. (1999) 340:803-811. The first such agent, a peptide termed "Peptide 3", was found to inhibit leukocyte migration induced by 5 different chemokines, while leaving migration in response to other chemoattractants (such as fMLP or TGF-beta) unaltered. This peptide, and its analogs such as NR58-3.14.3 (i.e. Sequence ID No.1 c(DCys-DGln-DIle-DTrp-DLys-DGln-DLys-DPro-DAsp-DLeu-DCys)-$NH_2$), are collectively termed "Broad Spectrum Chemokine inhibitors" (BSCIs). Grainger et al., Biochem. Pharm. 65 (2003) 1027-1034 have subsequently shown BSCIs to have potentially useful anti-inflammatory activity in a range of animal models of diseases. Interestingly, simultaneous blockade of multiple chemokines is not apparently associated with acute or chronic toxicity, suggesting this approach may be a useful strategy for developing new anti-inflammatory medications with similar benefits to steroids but with reduced side-effects.

However, peptides and peptoid derivatives such as NR58-3.14.3, may not be optimal for use in vivo. They are quite expensive to synthesise and have relatively unfavourable pharmacokinetic and pharmacodynamic properties. For example, NR58-3.14.3 is not orally bioavailable and is cleared from blood plasma with a half-life period of less than 30 minutes after intravenous injection.

Two parallel strategies have been adopted to identify novel preparations which retain the anti-inflammatory properties of peptide 3 and NR58-3.14.3, but have improved characteristics for use as pharmaceuticals. Firstly, a series of peptide analogs have been developed, some of which have longer plasma half-lives than NR58-3.14.3 and which are considerably cheaper to synthesise. Secondly, a detailed structure: activity analysis of the peptides has been carried out to identify the key pharmacophores and design small non-peptidic structures which retain the beneficial properties of the original peptide.

This second approach yielded several structurally distinct series of compounds which retained the anti-inflammatory properties of the peptides, including 16-amino and 16-aminoalkyl derivatives of the alkaloid yohimbine, as well as a range of N-substituted 3-aminoglutarimides. (Reference: Fox et al., J Med Chem 45(2002) 360-370: WO 99/12968 and WO 00/42071.) All of these compounds are broad-spectrum chemokine inhibitors which retain selectivity over non-chemokine chemoattractants, and a number of them have been shown to block acute inflammation in vivo.

The most potent and selective of these compounds was (S)-3-(undec-10-enoyl)-aminoglutarimide (NR58,4), which inhibited chemokine-induced migration in vitro with an $ED_{50}$ of 5nM. However, further studies revealed that the aminoglutarimide ring was susceptible to enzymatic ring opening in serum. Consequently, for some applications (for example, where the inflammation under treatment is chronic, such as in autoimmune diseases) these compounds may not have optimal properties, and a more stable compound with similar anti-inflammatory properties may be superior.

As an approach to identifying such stable anlogs, various derivatives of (S)- 3-(undec-10-enoyl)-aminoglutarimide have been tested for their stability in serum. One such derivative, the 6-deoxo analog (S)-3-(undec-10-enoyl)-tetrahydropyridin-2-one, is completely stable in human serum for at least 7 days at 37°C, but has considerably reduced potency compared with the parental molecule.

Amide derivatives of 3-aminocaprolactam have already been disclosed in the art. For example:

- Japanese patent application No. 09087331 describes 3-aminocaprolactam amide derivatives wherein the amide alkyl side chain may contain from 2 to 30 carbon atoms. These compounds have been presented as oil-gelating agents.
- US patent No. 6,395,282 describes immunogenic conjugates comprising a carrier molecule coupled to an autoinducer of a Gram negative bacteria, wherein said autoinducer can be a 3-aminocaprolactam amide derivative wherein the amide alkyl side chain may contain up to 34 carbon atoms. However, a therapeutic use is disclosed only for the

conjugates and not for the isolated amide derivative.

- An article by Weiss et al. (Research Communications in Psychology, Psychiatry and Behavior (1992), 17(3-4), 153-159) discloses a series of 3-aminocaprolactam amide derivatives, and among others 3-hexanamido-DL-ε-caprolactam and 3-dodecanamido-DL-ε-caprolactam. These compounds are presented as having only an *in vitro* activity but no significant *in vivo* effect.

In other words, though some alkyl amide derivatives of 3-aminocaprolactam have certainly been known in the art, no actual pharmaceutical use has been described for 3-aminocaprolactam amide derivatives.

The invention provides the use of a compound of general formula **(I)** or a pharmaceutically acceptable salt thereof in the preparation of a medicament for the treatment of an inflammatory disorder selected from the group consisting consisting of multiple sclerosis, stroke, coronary artery disease, myocardial infarction, unstable angina pectoris, atherosclerosis, vasculitis, viral infection or replication, asthma, osteoporosis, tumor growth, rheumatoid arthritis, organ transplant rejection, delayed graft or organ function, psoriasis, skin wounds, malaria, tuberculosis, allergies, Alzheimer's disease, immune response suppression, ALS, and fibrosis:

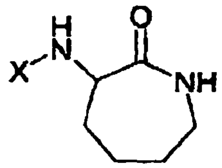

**(I)**

wherein

X is $-CO-R^1$ or $-SO_2-R^1$,

$R^1$ is an alkyl, haloalkyl, alkenyl or alkynyl radical of 4 to 20 carbon atoms which is di-substitated at the carbon of $R^1$ attached directly to the carbonyl carbon of X with the same or different groups selected from: alkyl, haloalkyl, alkoxy, haloalkoxy, alkenyl, alkynyl or alkylamino radicals, such that $R^1$ is 4 to 20 carbon atoms; and

$R^2$ is a linear or branched alkyl radical of 4 to 20 carbon atoms.

The carbon atom at position 3 of the caprolactam ring is asymmetric and consequently, the compounds according to the present invention have two possible enantiomeric forms, that is, the "R" and "S" configurations. The present invention encompasses the two enantiomeric forms and all combinations of these forms, including the racemic "RS" mixtures. With a view to simplicity, when no specific configuration is shown in the structural formulae, it should be understood that the two enantiomeric forms and their mixtures are represented.

GB priority applications 0327775.3 and 0417436.3 by the same applicant on 3-amino-caprolactam compounds have indicated correctly that 'S'-configuration compounds are preferred - these applications wrongly illustrate a general Formula (I') showing the 'R'-configuration.

Preferably, the compounds of general formula **(I)** or pharmaceutically acceptable salts thereof used according to this aspect of the invention will be compounds of general formula **(I')**

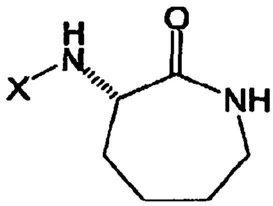

**(I')**

wherein X has the same meaning as above.

The invention also provides pharmaceutical compositions comprising, as active ingredient, a compound of general formula **(I)**, or a pharmaceutically acceptable salt thereof, and at least one pharmaceutically acceptable excipient and/or carrier:

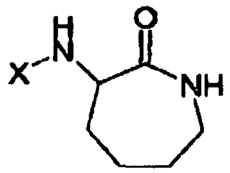

**(I)**

wherein
X is -CO-$R^1$ or -$SO_2$-$R^2$,
$R^1$ is an alkyl, haloalkyl, alkenyl or alkynyl radical of 4 to 20 carbon atoms which is di-substituted at the carbon of $R^1$ attached directly to the carbonyl carbon of X with the same or different groups selected from: alkyl, haloalkyl, alkoxy, haloalkoxy, alkenyl, alkynyl or alkylamino radicals, such that $R^1$ is 4 to 20 carbon atoms; and
$R^2$ is a linear or branched alkyl radical of 4 to 20 carbon atoms,

Preferably, the compounds of general formula **(I)** or pharmaceutically acceptable salts thereof used according to this aspect of the invention will be compounds of general formula **(I')**

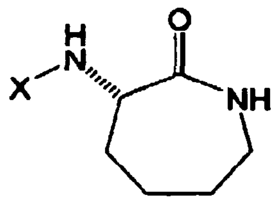

**(I')**

wherein X has the same meaning as above.

By pharmaceutically acceptable salt is meant in particular the addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Also within the scope of the present invention, when they can be used, are the salts formed from bases such as sodium or potassium hydroxide. For other examples of pharmaceutically acceptable salts, reference can be made to "Salt selection for basic drugs", Int. J. Pharm. (1986), 33, 201-217.

The pharmaceutical composition can be in the form of a solid, for example powders, granules, tablets, gelatin capsules, liposomes or suppositories. Appropriate solid supports can be, for example, calcium phosphate, magnesium stearate, talc, sugars, lactose, dextrin, starch, gelatin, cellulose, methyl cellulose, sodium carboxymethyl cellulose, polyvinylpyrrolidine and wax. Other appropriate pharmaceutically acceptable excipients and/or carriers will be known to those skilled in the art.

The pharmaceutical compositions according to the invention can also be presented in liquid form, for example, solutions, emulsions, suspensions or syrups. Appropriate liquid supports can be, for example, water, organic solvents such as glycerol or glycols, as well as their mixtures, in varying proportions, in water.

The invention also provides compounds and salts thereof of general formula (I)

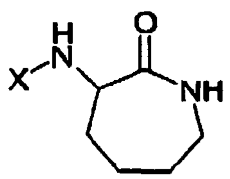

**(I)**

wherein
X is -CO-$R^1$ or -$SO_2$-$R^2$,
$R^1$ is an alkyl, haloalkyl, alkenyl or alkynyl radical of 4 to 20 carbon atoms which is di-substituted at the carbon of $R^1$ attached directly to the carbonyl carbon of X with the some or different groups selected from: alkyl, haloalkyl, alkoxy,

haloalkoxy, alkenyl, alkynyl or alkylamino radicals, such that $R^1$ is 4 to 20 carbon atoms; and
$R^2$ is a linear or branched alkyl radical of 4 to 20 carbon atoms.

Preferably, the compounds of general formula **(I)** or salts thereof used according to this aspect of the invention will be compounds of general formula **(I')**

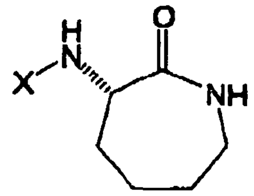

**(I')**

wherein X has the same meaning as above.

In particular, preferred compounds of general formula **(I)** or **(I')** and their salts according to any aspect of the present invention are selected from the group consisting of:

- (*S*)-3-(2',2'-dimethyl-dodecanoyl)amino-caprolactam;
- (*R*)-3-(2',2'-Dimethyl-dodecanoyl)amino-caprolactam;
- (*S*)-3-(2',2'-Dimethyl-pentanoyl)amino-caprolactam;
- (*S*)-3-(2',2'-Dimethyl-pent-4-enoyl)amino-caprolactam;
- (*S*)-3-(2',2'-Dimethyl-propionyl)amino-caprolactam;
- (*S*)-3-(2',2'-Dimethyl-butyryl)amino-caprolactam;
- (*S,E*)-3-(2',2'-Dimethyl-dodec-4'-enoyl)amino-caprolactam;
- (*S*)-3-(2',2',5'-Trimethyl-hex-4'-enoyl)amino-caprolactam;
- (*S'*)-3-(2',2',5'-Trimethyl-hexanoyl)amino-caprolactam;
- (*S*)-3-(Decanesnlfonyl)amino-caprolactam;
- (*S*)-3-(Dodecanesulfonyl)amino-caprolactam;
- (*S*)-3-(Tetradecanesu1fonyl)amino-caprolactam;
- (*S*)-3-(Hexadecanesulfonyl)amino-caprolactam;
- (*S*)-3-(Octadecanosalfonyl)amino-caprolactam ;
- (*S*)-(3'-Chloro-2'-(chloromethyl)-2'-methylpropionyl)amino-caprolactam; and pharmaceutically acceptable salts thereof.

The most preferred compounds will be selected from the group consisting of:

(S)-3-(2',2'-dimethyl-dodecanoyl)amino-caprolactam and (*S*)-3-(2',2'-dimethyl-propionyl)amino-caprolactam and salts thereof.

In compounds of general formula **(I)** or **(I')** according to any aspect of the present invention, the 4 to 20 carbon atoms comprise 5 to 20 carbon atoms, 8 to 20 carbon atoms, 9 to 20 carbon atoms, 10 to 18 carbon atoms, 12 to 18 carbon atoms, 13 to 18 carbon atoms, 14 to 18 carbon atoms, or 13 to 17 carbon atoms.

The invention includes compounds, compositions and uses thereof as defined, wherein the compound is in hydrated or solvated form.

The amide derivatives of 3-aminocaprolactam described here are functional BSCIs. They are relatively inexpensive to synthesise, using facile synthesis routes provided herein; they are stable in human serum and consequently have excellent pharmacokinetic properties; they are orally bioavailable; they are highly potent broad-spectrum chemokine inhibitors in vitro with excellent selectivity over non-chemokine chemoattractants; they are highly potent and effective anti-inflammatory agents in vivo in rodent models of inflammation; their administration is not associated with any significant acute toxicity at the doses necessary to achieve a maximal therapeutic effect. Taken together, these properties suggest that amide derivatives of 3-aminocaprolactam represent, anti-inflammatory medications with advantages over previously described compounds.

In comparison to the prior art the improvement of the present invention lies in the introduction of the aminocaprolactam moiety. However, the chemical structure of the side chain (whether alkyl amide or alkyl sulfonamide) can also significantly affect the properties of the molecule, such that alkyl substituents with substitution at the 2-position (relative to the amide carbonyl) or 1-position (relative to the sulfonamide sulfonyl group) are significantly superior to

compounds with linear alkyl chains (whether alkyl amides or alkyl sulfonamides).

Prior art peptides (such as NR58-3.14.3) have the disadvantages that: (a) they are expensive and require solid phase synthesis (at least for the longer ones) and (b) they clear very quickly via the kidneys and (c) they are generally less potent.

The prior art aminoglutarimides are cheap, not cleared quickly via the kidneys and more potent BUT they do not show metabolic stability.

The improvement described here, the uminocaprolactams, are cheap, not cleared by the kidney and even more potent, and are also metabolically stable.

Inflammatory disorders that can be prevented or treated by the compounds of general formula **(I)** or **(I')** or the pharmaceutically acceptable salts thereof or pharmaceutical compositions or medicaments containing them as active ingredients include notably:

- autoimmune diseases, for example such as multiple sclerosis;
- vascular disorders including stroke, coronary artery diseases, myocardial infarction, unstable angina pectoris, atherosclerosis or vasculitis, e. g., Behçet's syndrome, giant cell arteritis, polymyalgia rheumatica, Wegener's granulomatosis, Churg-Strauss syndrome vasculitis, Henoch-Schönlein purpura and Kawasaki disease;
- viral infection or replication, e.g. infections due to or replication of viruses including pox virus, herpes virus (e. g., Herpesvirus samiri), cytomegalovirus (CMV) or lentivirus;
- asthma;
- osteoporosis; (low bone mineral density);
- tumor growth;
- rheumatoid arthritis;
- organ transplant rejection and/or delayed graft or organ function, e.g. in renal transplant patients;
- a disorder characterised by an elevated TNF-$\alpha$ level;
- psoriasis;
- skin wounds;
- disorders caused by intracellular parasites such as malaria or tuberculosis;
- allergies; or
- Alzheimer's disease.

According to this invention, further inflammatory disorders include:

- ALS;
- fibrosis (particularly pulmonary fibrosis, but not limited to fibrosis in the lung);
- the formation of adhesions (particularly in the peritoneum and pelvic region).
- antigen induced recall response
- immune response suppression

These clinical indications fall under the general definition of inflammatory disorders or disorders characterized by elevated TNF$\alpha$ levels.

The invention also provides a compound of general formula **(I)** or **(I')** as defined herein for use in the treatment of an inflammatory disorder selected from the group consisting of multiple sclerosis, stroke, coronary artery disease, myocardial infarction, unstable angina pectoris, atherosclerosis, vasculitis, viral infection or replication, asthma, osteoporosis, tumor growth, rheumatoid arthritis, organ transplant rejection, delayed graft or organ function, psoriasis, skin wounds, malaria, tuberculosis, allergies, Alzheimer's disease, immune response, suppression, ALS, and fibrosis.

Administration of a medicament according to the invention can be carried out by topical, oral, parenteral route, by intramuscular injection, etc.

The administration dose envisaged for a medicament according to the invention is comprised between 0.1 mg and 10 g depending on the type of active compound used.

According to the invention, the compounds of general formula **(I)** or **(I')** can be prepared using the processes described hereafter.

<u>**Preparation of the compounds of general formula (I) or (I')**</u>

All the compounds of general formula **(I')** or **(I')** can be prepared easily according to general methods known to the person skilled in the art.

Nevertheless, the following preferred synthetic routes are proposed:

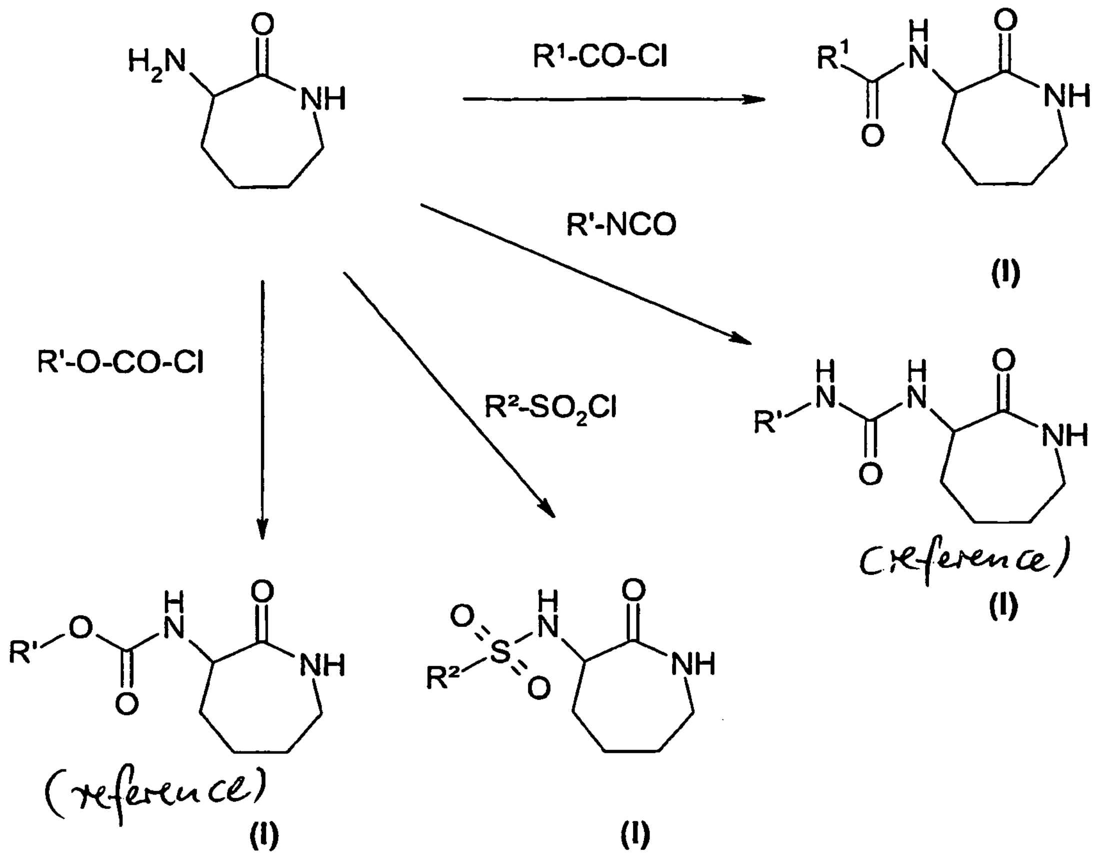


**Diagram 1**

According to the routes shown in Diagram 1:

- ❖ 3-amino-caprolactam is treated by an acid chloride of general formula $R^1$-CO-Cl wherein $R^1$ is an alkyl, haloalkyl, alkenyl or alkynyl radical to produce the compounds of general formula **(I)** wherein X is -CO-$R^1$ and $R^1$ is an alkyl, haloalkyl, alkenyl or alkynyl radical; or
- ❖ Reference only : 3-amino-caprolactam is treated by an isocyanate of general formula R'-NCO wherein R' is alkyl to produce the compounds of general formula **(I)** wherein X is -CO-$R^1$ and $R^1$ is an alkylamino radical ;
- ❖ 3-amino-caprolactam is treated by a sulphochloride of general formula $R^2$-$SO_2Cl$ wherein $R^2$ is alkyl to produce the compounds of general formula **(I)** wherein X is -$SO_2$-$R^2$ and $R^2$ is an alkyl radical ; or
- ❖ Reference only : 3-amino-caprolactam is treated by a chloroformate of general formula R'-O-CO-Cl wherein R' is alkyl to produce the compounds of general formula **(I)** wherein X is

  - CO-$R^1$ and $R^1$ is an alkoxy, radical.

The reactions shown in Diagram 1 may be carried out, for example, in chloroform or dichloromethane. The most preferred reaction solvent is dichloromethane.
The above reactions are preferably carried out in the presence of a base, for example $Na_2CO_3$.
All the above reactions may be carried out at ambient temperature (about 25 °C) or more generally at a temperature between 20 and 50 °C.

**DEFINITIONS**

The term "about" refers to an interval around the considered value. As used in this patent application, "about X" means an interval from X minus 10% of X to X plus 10% of X, and preferably an interval from X minus 5% of X to X plus 5% of X.
The use of a numerical range in this description is intended unambiguously to include within the scope of the

invention all individual integers within the range and all the combinations of upper and lower limit numbers within the broadest scope of the given range. Hence, for example, the range of 4 to 20 carbon atoms specified in respect of (*inter alia*) formula I is intended to include all integers between 4 and 20 and all sub-ranges of each combination of upper and lower numbers, whether exemplified explicitly or not.

As used herein, the term "comprising" is to be read as meaning both comprising and consisting of. Consequently, where the invention relates to a "pharmaceutical composition comprising as active ingredient" a compound, this terminology is intended to cover both compositions in which other active ingredients may be present and also compositions which consist only of one active ingredient as defined.

The term "peptidic moieties" used herein is intended to include the following 20 naturally-occurring proteogenic amino acid residues:

| SYMBOL | MEANING |
|---|---|
| Ala | Alanine |
| Cys | Cysteine |
| Asp | Aspartic Acid |
| Glu | Glutamic Acid |
| Phe | Phenylalanine |
| Gly | Glycine |
| His | Histidine |
| Ile | Isoleucine |
| Lys | Lysine |
| Leu | Leucine |
| Met | Methionine |
| Asn | Asparagine |
| Pro | Proline |
| Gln | Glutamine |
| Arg | Arginine |
| Ser | Serine |
| Thr | Threonine |
| Val | Valine |
| Trp | Tryptophan |
| Tyr | Tyrosine |

Unless otherwise defined, all the technical and scientific terms used here have the same meaning as that usually understood by an ordinary specialist in the field to which this invention belongs.

The following examples are presented in order to illustrate the above procedures.

**FIGURES**

Figure 1 provides a comparison of (R)-and (S)- enantiomers of amide derivatives of aminocaprolactam as inhibitors of MCP-1 induced migration.

## EXAMPLES

*General procedure for the synthesis of the starting compounds*

The hydrochlorides of (*R*) and (*S*)-3-amino-caprolactam, and the hydro-pyrrolidine-5-carhoxylates of (*R*,*R*) and (*S,S*)-3-amino-caprolactam were synthesised according to literature (cf. Boyle et al., J. Org. Chem., (1979), 44, 4841-4847; Rezler et al., J. Med. Chem. (1997), 40, 3508-3515).

**Reference Example 1: (*S*)-3-heaadecanoylamlao-caprolacCam:**

(*S*)-3-acnino-caprolactam hydrochloride (5 mmol) and $Na_2CO_3$ (15 mmol) in water (25 ml) were added to a solution of hexadecanoyl chloride (5 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo*. The residue was purified by recrystallisation from EtOAc to give the title compound (1.41 g; 77%).
Melting point: 99-100 °C. $[\alpha]_D^{23}$ (c=1, $CHCl_3$)=+32.0.
IR: $v_{max}$ ($cm^{-1}$): 3325, 3272 (NH), 1666, 1655, 1631 (CO), 1524 (NH).
$^1H$ NMR ($\delta_H$, 500 MHz, $CDCl_3$): 6.88 (1H, d, *J* 5.5, CHN*H*), 6.72 (1H, br s, $CH_2NH$), 4.49 (1H, ddd, *J* 11, 6, 1, C*H*NH), 3.29-3.16 (2H, m, $CH_2NH$), 2.17 (2H, t, *J* 7.5, $CH_2CONH$), 2.03 (1H, br d, *J* 13.5, ring CH), 1.98-1.89 (1H, m, ring CH), 1.85-1.73 (2H, m, ring CH), 1.58 (2H, br qn *J* 7.0, $CH_2CH_2CONH$), 1.43 (1H, br qd, *J* 14, 3, ring CH), 1.38-1.29 (1H, br m, ring CH), 1.29-1.14 (24H, m, $(CH_2)_{12}$) and 0.83 (3H, t. *J* 6.5, $CH_3$).
$^{13}C$ NMR ($\delta_c$, 125 MHz, $CDCl_3$): 175.9, 172.3 (CO), 52.0 (NHCHCO), 42.1 ($NCH_2$), 36.6, 1.9, 31.7, 29.6 (×6), 29.4, 293 (×2), 29.2, 28.8, 27.9, 25.6, 22.6 ($CH_2$) and
14.1 ($CH_3$).
m/z ($C_{22}H_{42}O_2Na$): 389.31450 (calculated: 389.3144).

**Reference Example 2: (*S*)-3-undecanoylamino-caprolactam:**

(*S*)-3-amino-caprolaatam hydrochloride (2 mmol) and $Na_2CO_3$ (6 mmol) in water (25 ml) were added to a solution of undecanoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo*. The residue was purified by recrystallisation from EtOAc to give the title compound (397 mg, 67%).
Melting point: 91-92 °C.

$[\alpha]_D^{23}$ (c=1,$CHCl_3$) = +30.2.

IR: $v_{max}$ ($cm^{-1}$): 3342, 3313 (NH), 1676, 1638 (CO), 1519 (NH); 3342, 3292 (NH), 1671, 1639 (CO), 1513 (NH).

$^1H$ NMR ($\delta_H$, 500 MHz, $d_6$-DMSO): 7.76 (1H, t, *J* 6, $CH_2NH$), 7.68 (1H, d, *J* 7, CHN*H*), 4.38 (1H, dd, *J* 10,7, CHNH), 3.15 (1H, ddd, *J* 15.5, 11, 5, CHHNH), 3.04 (1H, dt, *J* 13, 6, CH*H*NH), 2.19-2.06 (2H, m, $CH_2CONH$), 1.85 (1H, dt, *J* 10.5, 3, C-5 H), 1.77-1.68 (2H, m, C-4 H, C-6 H), 1.60 (1H, qt, *J* 12, 3.5, C-5 H), 1.46 (2H, br qn *J* 6.5, $CH_2CH_2CONH$), 1.35 (1H, qd, *J* 12.5, 3, C-4 H), 1.31-1.13 (15H, m, $(CH_2)_7$ + C-6 H) and 0.85 (3H, t, *J* 7.0, $CH_3$).

$^{13}C$ NMR ($\delta_c$, 125 MHz, $d_6$-DMSO): 174.4 (CO-ring), 171.3 (CO-chain), 51.3 (NHCHCO), 40.7 ($NCH_2$), 35.2, 31.4, 31.3, 29.1, 29.0 (×2), 28.9, 28.8, 28.7, 27.8, 25.4, 22.2 ($CH_2$) and 14.0 ($CH_3$).
m/z ($C_{17}H_{32}N_2O_2Na$): 319.23540 (calculated: 319.2361).

**Reference Example 3: (S)-3-(undec-10-enoyl)amino-caprolactam:**

(*S*)-3-amino-caprolactam hydrochloride (2 mmol) and $Na_2CO_3$ (6 mmol) in water (25 ml) were added to a solution of undec-10-enoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo*. The residue was purified by recrystallisation from EtOAc to give the title compound (423 mg; 72%).
Melting point: 83-84 °C.

$[\alpha]_D^{23}$ (c = 1, $CHCl_3$) = +40.1.

IR: $v_{max}$ ($cm^{-1}$): 3327, 3273 (NH), 1655, 1630 (CO), 1521 (NH).

$^1$H NMR ($\delta_H$, 500 MHz, $d_6$-DMSO): 7.75 (1H, t, *J* 6, $CH_2NH$), 7.66 (1H, d, *J* 7, CHN*H*), 5.76 (1H, ddt, *J* 17, 10, 6.5 $CH_2$=C*H*), 4.96 (1H, dq, *J* 17, 2, C*H*H=CH), 4.96 (1H, ddt, *J* 17, 2, 1, C*H*H=H), 4.36 (1H, dd, *J* 10, 7, C*H*NH), 3.14 (1H, ddd, *J* 15.5, 11.5, 5, C*H*HNH), 3.03 (1H, br dt, *J* 13, 5.5, CH*H*NH), 2.16-2.06 (2H, m, C$H_2$CONH), 1.98 (2H, br q, *J* 7, $CH_2$=CHC$H_2$), 1.85 (1H, dt, *J* 10.5, 3, C-5 H), 1.75-1.67 (2H, m, C-4 H, C-6 H), 1.60 (1H, qt, *J* 13,3.5, C-5 H), 1.44 (2H, br qn, *J* 7, C$H_2$C$H_2$CONH), 1.39-1.27 (3H, m, $CH_2$=CHC$H_2$C$H_2$ + C-4 H) and 1.31-1.13 (9H, m, $(CH_2)_4$ + C-6 H).

$^{13}$C NMR ($\delta_c$, 125 MHz, $d_6$-DMSO): 174.4 (CO-ring), 171.3 (CO-chain), 138.9 ($CH_2$=CH), 114.7 (*C*$H_2$-CH), 51.3 (NH-CHCO), 40.7 ($NCH_2$), 35.3, 33.3, 31.3,29.0,28.9 (×2) 28.7, 28.6, 28.4, 27.8 and 25.4 ($CH_2$).

m/z ($C_{17}H_{30}N_2O_2N_2Na$): 317.21970 (calculated: 317.2205).

**<u>Reference Example 4:</u> (*S*)-3-(undec-10-ynoyl)amino-caprolactam:**

(*S*)-3-amino-caprolactam hydrochloride (2 mmol) and $Na_2CO_3$ (6 mmol) in water (25 ml) were added to a solution of undec-10-ynoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by recrystallisation from EtOAc to give the title compound (362 mg; 62%).

Melting point: 73-75 °C.

$[\alpha]_D^{25}$ (c = 1, $CHCl_3$) = +42.1.

IR: $v_{max}$ ($cm^{-1}$): 3332, 3295 (NH), 1667, 1633 (CO), 1523 (NH).

$^1$H NMR ($\delta_H$, 500 MHz, $d_6$-DMSO): 7.76 (1H, t, *J* 5.5, $CH_2$N*H*), 7.68 (1H, d, *J* 7, CHNH), 4.36 (1H, dd, *J* 11, 7, C*H*NH), 3.16 (1H, ddd, *J* 15.5, 11.5, 5, C*H*HNH), 3.03 (1H, or dt, *J* 14, 7, CHHNH), 2.17-2.07 (4H, m, C$H_2$CONH + C$H_2$CCH), 1.85 (1H, m, C-5H), 1.77-1.67 (2H, m, C-4 H, C-6 H), 1.62 (1H, br qt, *J* 13, 3.0, C-5 H), 1.50-1.28 (5H, m, C$H_2$C$H_2$CONH + HCCC$H_2$C$H_2$ + C-4 H) and 1.28-1.13 (9H, m, $(CH_2)_4$ + C-6 H).

$^{13}$C NMR ($\delta_c$, 125 MHz, $d_6$-DMSO): 174.4 (CO-ring), 171.3 (CO-chain), 84.6 ($CH_2$*C*CH), 71.1 ($CH_2$C*C*H), 51.3 (NH*C*H-CO), 40.7 ($NCH_2$), 35.2, 31.3, 29.0, 28.8, 28.7, 28.5, 28.2, 28.0, 27.8, 25.4 and 17.8 ($CH_2$).

m/z ($C_{17}H_{28}N_2O_2Na$): 317.20470 (calculated: 315.2048).

**<u>Reference Example 5:</u> (*S*)-3-dodecanoylamino-caprolactam:**

(*S*)-3-amino-caprolactam hydrochloride (2 mmol) and $Na_2CO_3$ (6 mmol) in water (25 ml) were added to a solution of dodecanoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by recrystallisation from EtOAc to give the title compound (439 mg, 71%).

Melting point: 93-94 °C.

$[\alpha]_D^{25}$ (c = 1, $CHCl_3$) = +35.5.

IR: $\nu_{max}$ ($cm^{-1}$): 3324,3267 (NH), 1666, 1630 (CO), 1521 (NH).

$^1$H NMR ($\delta_H$, 500 MHz, $d_6$-DMSO): 7.76 (1H, br s, $CH_2$N*H*), 7.67 (1H, d, *J* 7, CHN*H*), 4.38 (1H, dd, *J* 10.5, 7.5, C*H*NH), 3.15 (1H, ddd, *J* 15.5, 11.5, 5, C*H*HNH), 3.05 (1H, dt, *J* 14.5, 5.5, CH*H*NH), 2.17-2.07 (2H, m, C$H_2$CONH), 1.90-1.80 (1H, m, C-5 H), 1.77-1.68 (2H, m, C-4 H, C-6 H), 1.62 (1H, br qt, *J* 12, 3.5, C-5 H), 1.46 (2H, br qn *J* 6.0, C$H_2$C$H_2$CONH), 1.36 (1H, qd, *J* 12.5, 2.5, C-4 H), 1.31-1.13 (17H, m, $(CH_2)_8$ + C-6 H) and 0.85 (3H, t, *J* 6.5, $CH_3$).

$^{13}$C NMR ($\delta_C$, 125 MHz, $d_6$-DMSO): 174.4 (CO-ring), 171.2 (CO-chain), 51.3 (NH*C*HCO), 40.7 ($NCH_2$), 35.3, 31.4, 31.3, 29.1 (×3), 29.0 (×2), 28.8, 28.7, 27.8, 25.4, 22.2 ($CH_2$) and 14.0 ($CH_3$).

m/z ($C_{16}H_{34}N_2O_2Na$): 333.25150 (calculated: 333.2518).

**<u>Reference Example 6;</u> (*S*)-3-tetradecanoylamino-caprolactam:**

(*S*)-3-amino-caprolactam hydrochloride (2 mmol) and $Na_2CO_3$ (6 mmol) in water (25 ml) were added to a solution of tetradecanoyl chloride (2 mmol) in dichloromethane (25 ml) at ambient temperature and the reaction mixture

was stirred for 2 hours. The organic layer was then separated and the aqueous phase was extracted with additional dichloramethane (2 × 25 ml). The combined organic layers were dried over $Na_2CO_3$ and reduced in *vacuo.* The residue was purified by recrystallisation from BtOAc to give the title compound (412 mg; 61 %).
Melting point: 97-98 °C.

$[\alpha]_D^{25}$ (c = 1, $CHCl_3$) = +33.2.

IR: $\nu_{max}$ ($cm^{-1}$): 3326, 3273 (NH), 1666, 1655, 1631 (CO), 1523 (NH).

$^1H$ NMR ($\delta_H$, 500 MHz, $CDCl_3$): 6.87 (1H, d, *J* 5.5, CHN*H*), 6.66-6.48 (1H, br m, $CH_2NH$), 4.50 (1H, dd, *J* 11, 6, C*H*NH), 3.30-3.16 (2H, m, C$H_2$NH), 2.18 (2H, t, *J* 7.5, C$H_2$CONH), 2.04 (1H, br d, *J* 13.5, ring CH), 2.00-1.92 (1H, m, ring CH), 1.86-1.74 (2H, m, ring CH), 1.59 (2H, br qn *J* 7.0, C$H_2$$CH_2$CONH), 1.43 (1H, br q, *J* 12.5, ring CH),1.31 (1H, br q, *J* 13, ring CH), 1.31-1.13 (20H, m, $(CH_2)_{10}$) and 0.85 (3H, t, *J* 6.5, $CH_3$).

$^{13}C$ NMR ($\delta_C$, 125 MHz, $CDCl_3$): 175.9, 172.3 (CO), 52.0 (NH*C*HCO), 42.1 ($NCH_2$), 36.6, 31.9, 31.7, 29.6 (×4), 29.4, 29.3 (×2), 29.2, 28.8, 27.9, 25.6, 22.6 ($CH_2$) and
14.1 ($CH_3$).
m/z ($C_{20}H_{38}N_2O_2Na$); 361.28270 (calculated: 361.2831).

**<u>Reference Example 7:</u> (*R*)-3-hexadecanoylamino-caprolactam:**

(*R,R*)-3-amino-caprolactam hydro-pyrrolidine-5-carboxylate (5 mmol) and $Na_2CO_3$ (15 mmol) in water (25 ml) were added to a solution of hexadecanoyl chloride (5 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by recrystallisation from EtOAc to give the title compound (1.23 g; 67%).
Melting point: 99-100 °C.

$[\alpha]_D^{20}$ (c = 1, $CHCl_3$) = -32.0.

**<u>Reference Example 8:</u> (*S*)-3-octadecanoylamino-caprolactum:**

(*S*)-3-amino-caprolactam hydrochloride (2 mmol) and $Na_2CO_3$ (6 mmol) in water (25 ml) were added to a solution of octadeconoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by recrystallisation from EtOAc to give the title compound (648 mg; 82%).
Melting point: 87-88 °C.

$[\alpha]_D^{25}$ (c = 1, $CHCl_3$) = +31.9.

IR: $\nu_{max}$ ($cm^{-1}$): 3327, 3272 (NH), 1667,1655, 1631 (CO), 1524 (NH).

$^1H$ NMR ($\delta_H$, 500 MHz, $CDCl_3$): 6.88 (1H, d, *J* 5.5, CHN*H*), 6.72-6.58 (1H, br m, $CH_2$N*H*), 4.50 (1H, dd, *J* 11, 6, C*H*NH), 3.29-3.16 (2H, m, C$H_2$NH), 2.17 (2H, t, *J* 7.5, C$H_2$CONH), 2.03 (1H, br d, *J* 13, ring CH), 1.99-1.90 (1H, m, ring CH), 1.86-1.73 (2H, m, ring CH), 1.58 (2H, br qn *J* 7.0, C$H_2$$CH_2$CONH), 1.42 (1H, br qd, *J* 14, 3, ring CH), 1.38-1.30 (1H, brm, ring CH), 1.30-1.14 (28H, m, $(CH_2)_{14}$) and 0.84 (3H, t, *J* 6.5, $CH_3$).

$^{13}C$ NMR ($\delta_C$, 125 MHz, $CDCl_3$): 175.9, 172.3 (CO), 52.0 (NH*C*HCO), 42.1 ($NCH_2$), 36.6, 31.9, 31.7, 29.6 (×8), 29.4, 29.3 (×2), 29.2, 28.8, 27.9, 25.6, 22.6 ($CH_2$) and
14.1 ($CH_3$).
m/z ($C_{24}H_{46}N_2O_2Na$): 417.34460 (calculated: 417.3457).

**<u>Reference Example 9:</u> (*S*)-(*Z*)-3-(hexadec-9-enoyl)amino-caprolactam:**

(*S,S*)-3-amino-caprolactam hydro-pyrrolidine-5-carboxylate (2 mmol) and $Na_2CO_3$ (6 mmol) in water (25 ml) were added to a solution of (*Z*)-hexadex-9-enoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and

reduced *in vacuo.* The residue was purified by silica column chromatography (eluent: EtOAc to 9:1 EtOAc:MeOH) to give the title compound (406 mg; 56%).
Melting point: 67-68 °C.

$[\alpha]_D^{23}$ (c = 1, $CHCl_3$) = +33.2.

IR: $\nu_{max}$ ($cm^{-1}$): 3324, 3268 (NH), 1655, 1630 (CO), 1524 (NH).

$^1$H NMR ($\delta_H$, 500 MHz, $CDCl_3$): 6.88 (1H, d, *J* 5.5, CHN*H*), 6.67 (1H, br s, $CH_2NH$), 5.33-5.25 (2H, m, C*H*=C*H*), 4.50 (1H, ddd, *J* 11, 6, 1, C*H*NH), 3.29-3.16 (2H, m, $CH_2NH$), 2.17 (2H, t, *J* 7.5, $CH_2CONH$), 2.03 (1 H, br d, *J* 13, ring CH), 1.99-1.90 (5H, m, ring CH + $CH_2CH$-$CHCH_2$), 1.84-1.72 (2H, m, ring CH), 1.58 (2H, br qn *J* 7.0, $CH_2CH_2CONH$), 1.43 (1H, br qd, *J* 14, 3, ring CH), 1.38-1.30 (1H, br m, ring CH), 1.30-1.14 (16H, m, $(CH_2)_4CH_2CH$=$HCH_2(CH_2)_4$) and 0.84 (3H, t, *J* 7, $CH_3$).

$^{13}$C NMR ($\delta_C$, 125 MHz, $CDCl_3$): 175.9, 172.3 (CO), 129.8 (×2) (CH=CH), 52.0 (NH*C*HCO), 42.0 ($NCH_2$), 36.6, 31.7 (×2), 29.7 (×2), 29.2 (×2), 29.1, 29.0, 28.8, 27.9, 27.2, 27.1, 25.6, 22.6 ($CH_2$) and 14.1 ($CH_3$).

m/z ($C_{22}H_{40}N_2O_2Na$): 387.29700 (calculated: 387.2987).

**<u>Reference Example 10:</u> (*S*)-(*Z*)-3-(octadec-9-enoyl)amino-caprolactam:**

(*S,S*)-3-amino-caprolactam hydro-pyrrolidine-5-carboxylate (2 mmol) and $Na_2CO_3$ (6 mmol) in water (25 ml) were added to a solution of (*Z*)-octadec-9-enoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (eluent: EtOAc to 9:1 EtOAc:MeOH) to give the title compound (514 mg; 66%).
Melting point: 66-67 °C.

$[\alpha]_D^{23}$ (c = 1, $CHCl_3$) = +30.9.

IR: $\nu_{max}$ ($cm^{-1}$): 3327, 3268 (NH), 1655, 1631 (CO), 1523 (NH).

$^1$H NMR ($\delta_H$, 500 MHz, $CDCl_3$): 6.88 (1H, d, *J* 5.5, CHN*H*), 6.74 (1H, br t, *J* 5, $CH_2NH$), 5.33-5.24 (2H, m, C*H*=C*H*), 4.49 (1H, ddd, *J* 11, 6, 1.5, C*H*NH), 3.29-3.14 (2H, m, $CH_2NH$), 2.16 (2H, t, *J* 7.5, $CH_2CONH$), 2.03 (1H, br d, *J* 13.5, ring CH), 1.99-1.89 (5H, m, ring CH + $CH_2CH$=$CHCH_2$), 1.84-1.72 (2H, m, ring CH),
1.58 (2H, br qn *J*7.0, $CH_2CH_2CONH$), 1.42 (1H, br qd, *J* 14, 3, ring CH), 1.38-1.30 (1H, br m, ring CH), 1.30-1.14 (20H, m, $(CH_2)_6CH_2CH$=$CHCH_2(CH_2)_4$) and 0.83 (3H t, *J* 7, $CH_3$).

$^{13}$C NMR ($\delta_C$, 125 MHz, $CDCl_3$): 175.9, 172.3 (CO), 129.9. 129.7 (CH=CH), 52.0 (NH*C*HCO), 42.0 ($NCH_2$), 36.6, 31.8, 31.7, 29.7 (×2), 29.5, 29.3 (×3), 29.2, 29.1, 28.8, 27.9, 27.2, 27.1, 25.6, 22.6 ($CH_2$) and 14.1 ($CH_3$).

m/z ($C_{24}H_{44}N_2O_2Na$): 415.32820 (calculated: 415.3300).

**<u>Reference Example 11:</u> (*R*)-(*Z*)-3-(octadec-9-enoyl)amino-caprolactam:**

(*R,R*)-3-amino-caprolactam hydro-pyrrolidine-5-carboxylate (2 mmol) and $Na_2CO_3$ (6 mmol) in water (25 ml) were added to a solution of (*Z*)-octadec-9-enoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (eluent: EtOAc to 9:1 EtOAc:MeOH) to give the title compound (574 mg; 73%).
Melting point: 66-67 °C.

$[\alpha]_D^{23}$ (c = 1, $CHCl_3$) = -31.4.

**<u>Example 1:</u> (*S*)-3-(2',2'-dimethyl-dodecanoyl)amino-caprolactam:**

(*S,S*)-3-amino-caprolactam hydro-pymolidine-5-carboxylate (2 mmol) and $Na_2CO_3$ (6 mmol) in water (25 ml) were added to a solution of 2,2-dimethyl-dodecanoyl chloride (2 mmol) in dichloromethane (25 ml) at ambient temperature and the reaction mixture was stirred for 2 hours. The organic layer was then separated and the aqueous phase was

extracted with additional dichloromethane (2 × 25 ml). The combined organic layers were dried over $Na_2CO_3$ and reduced in *vacuo.* The residue was purified by silica column chromatography (eluent: EtOAc to 9:1 EtOAc:MeOH) to give the title compound (543 mg; 80%).
Melting point: 41-42 °C.

$[\alpha]_D^{25}$ (c = 1, $CHCl_3$) = +28.0.

IR: $\nu_{max}$ ($cm^{-1}$): 3403, 3265 (NH), 1673, 1641 (CO), 1497 (NH).

$^1H$ NMR ($\delta_H$, 500 MHz, $CDCl_3$): 7.08 (1H, d, *J* 5.5, CHN*H*), 6.67 (1H, br s, $CH_2NH$), 4.44 (1H, dd, *J* 11, 5.5, C*H*NH), 3.28-3.15 (2H, m, $CH_2NH$), 2.01 (1H, br d, *J* 13, ring CH), 1.98-1.89 (1H, m, ring CH), 1.84-1.72 (2H, m, ring CH), 1.47-1.30 (3H, br m, ring CH + $CH_2CMe_2CONH$), 1.27-1.15 (17H, br m, ring CH +$(CH_2)_8$) 1.13 (3H, 8, C*Me*Me), 1.12 (3H, s, CMe*Me*) and 0.82 (3H, t, *J* 7, $CH_2CH_3$).

$^{13}C$ NMR ($\delta_C$, 125 MHz, $CDCl_3$): 177.1, 176.0 (CO), 52.0 (NH*C*HCO), 41.9 ($CMe_2$), 42.1, 41.3, 31.8, 31.5, 30.1, 29.6, 29.5 (×2), 29.3, 28.9, 27.9 ($CH_2$), 25.3, 25.2 ($CH_3$), 24.8, 22.6 ($CH_2$) and 14.1 ($CH_3$).

m/z ($C_{20}H_{38}N_2O_2Na$): 361.28350 (calculated: 361.2831).

Compound 12 was later resynthesised on a larger scale, and this batch of material had the following properties: melting point 51-52 °C. $[\alpha]_D^{25}$ (c = 1, $CHCl_3$) +28.0; $[\alpha]_D^{25}$ (c = 0.87, MeOH) +13.3.

**<u>Reference Example 12:</u> (*S*)-3-(decyloxytorbonyl)amino-caprolactam:**

(*S,S*)-3-amino-caprolactam hydro-pyrrolidine-5-carboxylate (2 mmol) and $Na_2CO_3$ (6 mmol) in water (25 ml) were added to a solution of decyl chloroformate (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (eluent: EtOAc to 9:1 EtOAc:MeOH) to give the title compound (459 mg; 74%).
Melting point: 40-41 °C.

$[\alpha]_D^{25}$ (c = 1, $CHCl_3$) = +31.4.

IR: $\nu_{max}$ ($cm^{-1}$): 3352, 3300 (NH), 1682, 1657, 1637 (CO), 1513 (NH).

$^1H$ NMR ($\delta_H$, 500 MHz, $CDCl_3$): 6.86 (1H, br s, $CH_2NH$), 6.72 (1H, d, *J* 6 CHN*H*), 4.49 (1H, dd, *J* 11, 6, C*H*NH), 3.99 (2H, t, *J* 6, $OCH_2$), 3.26-3.14 (2H, m, $CH_2NH$), 2.04 (1H, br d, *J* 13.5, ring CH), 2.00-1.91 (1H, m, ring CH), 1.82-1.68 (2H, m, ring CH), 1.55 (2H, br qn *J* 7.0, $CH_2CH_2O$), 1.48 (1H, br qd, *J* 14, 2.5, ring CH), 1.38-1.31 (1H, br m, ring CH), 1.29-1.17 (14H, m, $(CH_2)_7$) and 0.83 (3H, t, *J* 7, $CH_3$).

$^{13}C$ NMR ($\delta_c$, 125 MHz, $CDCl_3$): 175.8, 55.9 (CO), 65.0 ($OCH_2$), 53.5 (NH*C*HCO), 42.0 ($NCH_2$), 32.1, 31.8, 29.5 (×2), 29.2 (×2), 29.0, 28.8, 28,0, 25.8, 22.6 ($CH_2$) and 14.1 ($CH_3$).

m/z ($C_{17}H_{32}N_2O_3Na$): 335.23190 (calculated: 335.2311).

**<u>Reference Example 13:</u> (*S*)-(*E*)-3-(dodec-2-enoyl)amino-caprolactam:**

(*S,S*)-3-amino-coprolactam hydro-pyrrolidine-5-carboxylate (2 mmol) and $Na_2CO_3$ (6 mmol) in water (25 ml) were added to a solution of dodec-2-enoyl chloride (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (eluent: EtOAc to 9:1 EtOAc:MeOH) to give the title compound (472 mg; 77%).
Melting point: 87-88 °C.

$[\alpha]_D^{25}$ (c = 1, $CHCl_3$) = +44.7.

IR: $\nu_{max}$ ($cm^{-1}$): 3382, 3331 (NH), 1660, 1616 (CO), 1520 (NH).

$^1H$ NMR ($\delta_H$, 500 MHz, $CDCl_3$): 6.94 (1H, d, *J* 5.5, CHN*H*), 6.84 (1H, br s, $CH_2NH$), 6.78 (1H, dt, *J* 15.5, 7, $CH_2CH$=CH), 5.80 (1H, d, *J* 15.5, $CH_2CH$=C*H*), 4.56 (1H, ddd, *J* 11, 6,1.5, C*H*NH), 3.29-3.15 (2H, m, $CH_2NH$), 2.11 (2H, q, *J* 7, $CH_2CH$=CH),

2.07 (1H, br d, *J* 13.5, ring CH), 1.98-1.90 (1H, m, ring CH), 1.86-1.73 (2H, m, ring CH), 1.44 (1H, br qd, *J* 14, 2.5, ring CH), 1.41-129 (3H, br m, ring CH + C*$H_2$*$CH_2$CH=CH), 1.29-1.14 (12H, m, $(CH_2)_6$) and 0.82 (3H, t, *J* 6.5, $CH_3$).

$^{13}$C NMR ($\delta_C$, 125 MHz, $CDCl_3$): 175.9, 165.0 (CO), 144.8, 123.5 (CH=CH), 52.0 (NH*C*HCO), 42.0 ($NCH_2$), 32.0, 31.8, 31.6, 29.4 (×2), 29.2, 29.1, 28.8, 28.2, 27.9, 22.6 ($CH_2$) and 14.1 ($CH_3$).

m/z ($C_{18}H_{31}N_2O_2Na$): 331.23570 (calculated: 331.2361).

**<u>Reference Example 14:</u> (*S*)-3-(dec-9-enylaminocarbonyl)amino-caprolactam:**

(*S,S*)-3-amino-caprolactam hydro-pyrrolidine-5-carboxylate (2 mmol) and $Na_2CO_3$ (6 mmol) in water (25 ml) were added to a solution of dec-9-enyl isocyanate (2 mmol) in dichloromethane (25 ml) at ambient temperature and the reaction mixture was stirred for 2 hours. The organic layer was then separated and the aqueous phase was extracted with additional dichloromethane (2 × 25 ml). The combined organic layers were dried over $Na_2CO_3$ and reduced in vacuo. The residue was purified by silica column chromatography (eluent: EtOAc to 9:1 EtOAc:MeOH) to give the title compound (347 mg; 56%).
Melting point: 98-99 °C.

$[\alpha]_D^{25}$ (c = 1, $CHCl_3$) = +27.3.

IR: $\nu_{max}$ ($cm^{-1}$): 3365, 3327, 3276 (NH), 1619, (CO), 1551 (NH).

$^1$H NMR ($\delta_H$, 500 MHz, $CDCl_3$): 6.64 (1H, br s, ring $CH_2$N*H*), 6.12 (1H, d, *J*6 CHN*H*), 5.75 (1H, ddtd, *J* 17, 10, 6.5, 1.5, $CH_2$=C*H*), 5.21-5.12 (1H, br m, urea $CH_2$NH).

4.93 (1H, dq, *J* 17, 1.5, CH*H*=CH), 4.87 (1H, br d, *J* 10, CH*H*=CH), 4.49 (1H, dd, *J* 11, 6,NHC*H*CO), 3.25(1H, ddd, *J* 15.5, 12, 4, ring $CH_2$N), 3.17 (1H, dt, *J* 14, 6, ring $CH_2$N), 3.11-3.02 (2H, m, urea NHC*$H_2$*), 2.05-1.87 (4H, br m, ring CH ×2 + C*$H_2$*CH-CH). 1.82-1.70 (2H, m, ring CH),1.48-1.36 (3H, br m, chain C*$H_2$*$CH_2$NH, + ring CH), 1.36-1.27 (3H, m, ring CH + chain $CH_2$) and 1.27-1.17 (8H, m, chain $(CH_2)_4$).

$^{13}$C NMR ($8_c$, 125 MHz, $CDCl_3$): 177.2, 157.6 (CO), 139.1,114.1 (CH=CH), 52.7 (NHCHCO), 42.1, 40.3 ($NCH_2$), 33.7, 32.9, 30.3, 29.4, 29.3, 29.0, 28.8 (×2), 27.9 and 26.9 ($CH_2$).

m/z ($C_{17}H_{31}N_3O_2Na$): 332.23150 (calculated: 332.2314).

**<u>Reference Example 15:</u> (*S*)-3-(decylaminocarbonyl)amino-caprolactam:**

(*S,S*)-3-amino-caprolactam hydro-pyrrolidine-5-carboxylate (2 mmol) and $Na_2CO_3$ (6 mmol) in water (25 ml) were added to a solution of decyl isocyanate (2 mmol) in dichloromethane (25 ml) at ambient temperature and the reaction mixture was stirred for 2 hours. The organic layer was then separated and the aqueous phase was extracted with additional diohlorometltane (2 × 25 ml). The combined organic layers were dried over $Na_2CO_3$ and reduced *in vacuo.* The residue was purified by silica column chromatography (eluent: EtOAc to 9:1 EtOAc:MeOH) to give the title compound (401 mg, 64%).
Melting point: 97-98 °C.

$[\alpha]_D^{25}$ (c = 1, $CHCl_3$) = +27.7.

IR: $v_{max}$ ($cm^{-1}$): 3359,3316 (NH), 1621, (CO), 1558 (NH).

$^1$H NMR ($\delta_H$, 500 MHz, $CDCl_3$): 6.62 (1 H, br s, ring $CH_2$N*H*), 6.09 (1H, d, *J* 6 CHN*H*), 5.16 (1H, br t, *J* 5, urea $CH_2$NH), 4.48 (1H, ddd, *J* 11, 6, 1, NHC*H*CO), 3.26 (1H, ddd, *J* 16, 11, 5, ring $CH_2$N), 3.17 (1H, dt, *J* 15, 7, ring $CH_2$N), 3.11-3.02 (2H, m, urea NHC*$H_2$*), 2.02 (1H, br d *J* 14, ring CH), 1.96-1.87 (1H, m, ring CH), 1.83-1.70 (2H, m, ring CH), 1.48-1.27 (4H, br m, ring CH ×2 + chain $CH_2$), 1.27-1.14 (14H, m, $(CH_2)_7$) and 0.82 (3H, t, *J* 7, $CH_3$).

$^{13}$C NMR ($\delta_c$,125 MHz, $CDCl_3$): 177.2, 157.6 (CO), 52.7 (NH*C*HCO), 42.1, 40.4 ($NCH_2$), 32.9, 31.8, 30.2, 29.6, 29.5, 29.4, 29.3, 28.8, 27.9, 26.9, 22.6 ($CH_2$) and 14.1.

m/z ($C_{17}H_{33}N_3O_2Na$): 334.24880 (calculated: 334.2470).

**<u>Example 2:</u> (*R*)-3-(2',2'-Dimethyl-dodecanoyl)amino-caprolactam:**

(*R,R*)-3-amino-caprolactam hydro-pyrrolidine-5-carboxylate (2 mmol) and $Na_2CO_3$ (6 mmol) in water (25 ml) were added to a solution of 2,2-dimethyl-dodecanoyl chloride (2 mmol) in dichloromethane (25 ml) at ambient temperature and the reaction was stirred for 2 hours. The organic layer was then separated and the aqueous phase was extracted

with additional dichloromethane (2 × 25 ml). The combined organic layers were dried over $Na_2CO_3$ and reduced *in vacuo.* The residue was purified by silica column chromatography (EtOAc: hexanes 1:3 to EtOAc) to give (*R*)-3-(2',2'-dimethyl-dodecanoyl)amino-caprolactam (515 mg, 76%); m.p. 48-49 °C; $[\alpha]_D^{25}$ (c = 1, $CHCl_3$) -25.7; $[\alpha]_D^{25}$ (c = 0.5, MeOH) -12.2.

Compound 17 was later resynthesised on a larger scale, and this batch of material had the following properties: melting point 50-51 °C.

**<u>Example 3:</u> (*S*)-3-(2',2'-Dimethyl-pentanoyl)amino-caprolactam:**

(*S,S*)-3-amino-caprolactam hydro-pyrrolidine-5-carboxylate (20 mmol) and $Na_2CO_3$ (60 mmol) in water (50 ml) were added to a solution of 2,2-dimethyl-pentanoyl chloride (20 mmol) in dichloromethane (50 ml) at ambient temperature and the reaction was stirred for 12 hours. The organic layer was then separated and the aqueous phase was extracted with additional dichloromethane (2 × 25 ml). The combined organic layers were dried over $Na_2CO_3$ and reduced in *vacuo.* The residue was recrystallised from EtOAc / hexane to give **(*S*)-3-(2',2'-dimethyl-pentanoyl)amino-caprolactam** (3.50 g, 77%); m.p. 84-85 °C; $[\alpha]_D^{25}$ (c=1, $CHCl_3$) +30.7; $\nu_{max}/cm^{-1}$ 3387, 3239 (NH), 1655,1634 (CO), 1507 (NH); $\delta_H$ (500 MHz, $CDCl_3$) 7.08 (1H, d, *J* 5, CHN*H*), 6.53 (1H, br s, $CH_2$N*H*), 4.45 (1H, ddd, *J* 11, 5.5, 1.5, C*H*NH), 3.29-3.16 (2H, m, C$H_2$NH), 2.00 (1H, br d, *J*13, ring CH), 1.98-1.92 (1H, m, ring CH), 1.84-1.73 (2H, m, ring CH), 1.47-1.30 (4H, br m, ring CH ×2 + C$H_2$C$Me_2$CONH), 1.23-1.15 (2H, m, C$H_2$$CH_3$) 1.14 (3H, s, C*Me*Me),1.13 (3H, s. CMe*Me*) and 0.84 (3H, t, *J*7, $CH_2$C$H_3$); $\delta_c$ (125 MHz, $CDCl_3$) 177.0, 176.1 (CO), 52.1 (NHCHCO), 43.6, 42.0 (×2, one of which is *C*$Me_2$), 31.5, 28.9, 27.9 ($CH_2$), 25.3, 25.2 ($CH_3$), 18.0 ($CH_2$) and 14.5 ($CH_3$); m/z ($M^+$ $C_{13}H_{24}N_2O_2$ requires 240.18378) 240.18437.

**<u>Example 4:</u> (*S*)-3-(2',2'-Dimethyl-pent-4-enoyl)amino-caprolactam:**

(*S*,S)-3-amino-caprolactan hydro-pyirolidine-5-carboxylate (20 mmol) and $Na_2CO_3$ (60 mmol) in water (50 ml) were added to a solution of 2,2-dimethyl-pent-4-enoyl chloride (20 mmol) in dichloromethane (50 ml) at ambient temperature and the reaction was stirred for 2 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (1:1 EtOAc: hexane to EtOAc) to give **(*S*)-3-(2',2-dimethyl-pent-4-enoyl)amino-caprolactam** (1.43 g, 32%); m.p. 71-72 °C; $[\alpha]_D^{25}$ (c = 1, $CHCl_3$) +27.7; $\nu_{max}/cm^{-1}$ 3395, 3304 (NH),1675, 1633 (CO), 1534 (NH); $\delta_H$ (500 MHz, $CDCl_3$) 7.10 (1H, d, J 4.5, CHN*H*), 6.48 (1H, br s, $CH_2$N*H*), 5.68 (1H, ddt, *J* 17, 10, 7.5, C*H*=$CH_2$), 5.02 (1H, br d, *J* 17 CH=C*H*H), 5.00 (1H, br d, *J* 10, CH=CH*H*), 4.45 (1H, dd, *J* 11, 5.5, C*H*NH), 3.30-3.17 (2H, m, C$H_2$NH), 2.27 (1H, *J* 14,7.5, C*H*HCH-$CH_2$), 2.22 (1H, dd, *J* 14, 7.5, CH*H*CH=$CH_2$), 2.01 (1H, br d, *J* 13, ring CH), 1.98-1.92 (1H, m, ring CH), 1.85-1.73 (2H, m, ring CH), 1.47-1.30 (2H, br m, ring CH ×2), 1.16 (3H, s, C*Me*Me) and 1.15 (3H, s, CMe*Me*); $\delta_c$ (125 MHz, $CDCl_3$) 176.4, 175.9 (CO), 134.2 (*C*H=$CH_2$), 117.8 (CH=*C*$H_2$), 52.1 (NH*C*HCO), 45.2, 42.1 ($CH_2$), 41.9 (*C*$Me_2$), 31.5, 28.9, 27.9 ($CH_2$), 25.0 and 24.9 ($CH_3$); m/z ($M^+$ $C_{13}H_{22}N_2O_2$ requires 238.16813) 238.16834.

**<u>Example 5:</u> (*S*)-3-(2',2'-Dimethyl-propionyl)amino-caprolactam:**

(*S,S*)-3-amino-caprolactam hydro-pyrrolidine-5-carboxylate (5 mmol) and $Na_2CO_3$ (15 mmol) in water (15 ml) were added to a solution of 2,2-dimethyl-propionyl chloride (5 mmol) in dichloromethane (15 ml) at ambient temperature and the reaction was stirred for 12 hours. The organic layer was then separated and the aqueous phase was extracted with additional dichloromethane (2 × 25 ml). The combined organic layers were dried over $Na_2SO_4$ and reduced *in vacuo.* The residue was recrystallised from EtOAc / hexane to give **(*S*)-3-(2',2'-dimethyl-propionyl)amino-caprolactam** (645 mg, 61%); m.p. 126-127 °C; $[\alpha]_D^{25}$ (c - 1, $CHCl_3$) +39.5; $\nu_{max}/cm^{-1}$ 3381, 3255 (NH), 1680,1632 (CO), 1506 (NH); $\delta_H$ (500 MHz, $CDCl_3$) 7.10 (1H, d, *J* 5.0, CHN*H*), 6.75 (1H, br s, $CH_2$N*H*), 4.42 (1H, ddd, *J* 11, 5.5, 1.5, C*H*NH), 3.27-3.16 (2H, m, C$H_2$NH), 2.03-1.89 (2% m, 2 × ring CH), 1.83-1.71 (2H, m, 2 × ring CH), 1.45-1.28 (2H, m, 2 × ring CH) and 1.15 (9H, s, 3 × $CH_3$); $\delta_c$ (125 MHz, $CDCl_3$) 177.7, 176.1 (CO), 52.1 (NH*C*HCO), 42.0 ($CH_2$N), 40.5 (*C*CO), 31.5, 28.9, 27.9 ($CH_2$ lactam), 27.4 (3 × $CH_3$).

m/z ($MNa^+$ $C_{11}H_{20}N_2O_2Na$ requires 235.141699) 235.142237; ($MH^+$ $C_{11}H_{21}N_2O_2$ requires 213.1597543) 213.160246.

**Example 6: (*S*)-3-(2',2'-Diabethyl-butyryl)amino-caprolactam:**

(*S,S*)-3-amino-caprolactam hydro-pyrrolidine-5-carboxylate (5 mmol) and $Na_2CO_3$ (15 mmol) in water (15 ml) were added to a solution of 2,2-dimethyl-butyryl chloride (5 mmol) in dichloromethane (15 ml) at ambient temperature and the reaction was stirred for 12 hours. The organic layer was then separated and the aqueous phase was extracted with additional dichloromethane (2 × 25 ml). The combined organic layers were dried over $Na_2SO_4$ and reduced *in vacuo.* The residue was recrystallised from EtOAc / hexane to give **(*S*)-3-(2',2'-dimethyl-butyryl)amino-caprolactam** (562 mg, 50%); m.p. 106-107 °C; $[\alpha]_D^{25}$ (c =1, $CHCl_3$) +33.6; $\nu_{max}/cm^{-1}$ 3400, 3278 (NH), 1677, 1630 (CO), 1500 (NH); $\delta_H$ (500 MHz, $CDCl_3$) 7.08 (1H, d, *J* 5.0, CHN*H*), 6.72 (1H, br s, $CH_2NH$), 4.44 (1H, ddd, *J* 11, 5,5, 1.5, C*H*NH), 3.28-3.16 (2H, m, $CH_2NH$), 2.04-1.90 (2H, m, 2 × ring CH), 1.83-1.72 (2H, m, 2 × ring CH), 1.57-1.44 (2H, m, $CH_2CH_3$), 1.44-1.30 (2H, m, 2 × ring CH) 1.12 (3H, s, $CH_3$) 1.11 (3H, s, $CH_3$) and 0.78 (3H, t, *J* 7.5, $CH_2CH_3$); $\delta_c$ (I25 MHz, $CDCl_3$) 177.0, 176.0 (CO), 52.1 (NH*C*HCO), 42.2 (*C*CO), 42.0 ($CH_2N$), 33.7 ($CH_2CH_3$), 31.6, 28.9, 27.9 ($CH_2$ lactam), 24.8, 24.7 ($CCH_3$) and 9.1 ($CH_2CH_3$); m/z ($MH^+$ $C_{12}H_{13}N_2O_2$ requires 227.1760) 227.1767.

**Example 7: (*S,E*)-3-(2',2'-Dimethyl-dodec-4'-enoyl)amino-caprolactam:**

(*S,S*)-3-amino-caprolactam hydro-pyrrolidine-5-carboxylate (10 mmol) and $Na_2CO_3$ (30 mmol) in water (30 ml) were added to a solution of 2,2-dimethyl-dodec-2-enoyl chloride (crude, from above reaction) (10 mmol) in dichloromethane (30 ml) at ambient temperature and the reaction was stirred for 12 hours. The organic layer was then separated and the aqueous phase was extracted with additional dichlonomethane (2 × 25 ml). The combined organic layers were dried over $Na_2CO_3$ and reduced *in vacuo.* The residue was purified by silica column chromatography (1:1 EtOAc: hexanes to EtOAc) to give (*S,E*)-3-(2',2'-dimethyl-dodec-4'-enoyl)amino-caprolactam as a colourless oil (2.12 g, 63%); $[\alpha]_D^{25}$ (c=1, $CHCl_3$) +21.6; $v_{max}/cm^{-1}$ 3264 (NH), 1639 (CO), 1497 (NH); $\delta_H$ (500 MHz, $CDCl_3$) 7.09 (1H, d, *J* 5.5, CHN*H*), 6.67-6.32 (1H, br m, $CH_2NH$), 5.42 (1H, dt, *J* 15, 6.5, C*H*=CH), 5.28 (1H, dt, *J* 15, 7, CH=C*H*), 4.44 (1H, dd, *J* 11, 5.5, C*H*NH), 3.30-3.17 (2H, m, $CH_2NH$), 2.20 (1H, dd, 13.5, 7, CH=CH$CH_2$), 2.14 (1H, dd, 13.5,7, CH=CH$CH_2$), 2.01-1.87 (4H, br m, ring CH ×2, + $CH_2$CH=CH), 1.87-1.74 (2m m, ring CH), 1.47-1.32 (2H, m, ring CH), 1.27-1.15 (10H, br m, $(CH_2)_3$) 1.13 (3H, s, C*Me*Me), 1.12 (3H, s, CMe*Me*) and 0.83 (3H, t, *J* 7, $CH_2CH_3$); $\delta_c$ (125 MHz, $CDCl_3$) 176.8,176.0 (CO), 134.2, 125.2 (CH=CH), 52.1 (NH*C*HCO), 43.9 ($CH_2$), 42.1 (×2) ($CH_2$ + *C*$Me_2$), 32.6, 31.8, 31.5, 30.1, 29.4, 29.1 (×2), 28.9,27.9 ($CH_2$), 25.0, 24.8 ($CH_3$) and 22.6 ($CH_3$); m/z ($MH^+$ $C_{20}H_{37}N_2O_2$ requires 337.2855) 337.2858.

**Example 8: (*S*)-3-(2',2',5'-Trimethyl-hex-4'-enoyl)amino-caprolactam:**

(*S,S*)-3-amino-caprolactam hydro-pyrrolidine-5-carboxylate (4.11 g, 16 mmol) and $Na_2CO_3$ (5.09 g, 48 mmol) in water (50 ml) were added to a solution of 2,2,5-trimethyl-hex-4-enoyl chloride (16 mmol) in dichloromethane (50 ml) at ambient temperature and the reaction was stirred for 12 hours. The organic layer was then separated and the aqueous phase was extracted with additional dichloromethane (2 × 50 ml). The combined organic layers were dried over $Na_2CO_3$ and reduced *in vacuo.* The residue was purified by silica column chromatography (1:5 EtOAc: hexanes to EtOAc) to give (*S*)-3-(2',2',5'-trimethyl-hex-4'-enoyl)amino-caprolactam as a waxy solid (3.58 g, 84%); m.p. 43-44 °C; $[\alpha]_D^{25}$ (c = 1, $CHCl_3$) +23.2; $\nu_{max}/cm^{-1}$ 3394, 3251 (NH), 1674, 1633 (CO), 1503 (NH); $\delta_H$ (500 MHz, $CDCl_3$) 7.11 (1H, d, *J* 5.0, CHN*H*), 6.65-6.45 (1H, br m, $CH_2NH$), 5.04 (1H, t, *J* 7.5, CH=C), 4.44 (1H, ddd, *J* 11, 5.5, 1.5, C*H*NH), 3.24-3.16 (2H, m, $CH_2NH$), 2.20 (1H, dd, *J* 14.5, 7.5, C=CH$CH_2$), 2.15 (1H, dd, *J,* 14.5, 7.5, C=CH$CH_2$), 2.03-1.90 (2H, m, 2 × ring CH), 1.84-1.72 (2H, m, 2 × ring CH), 1.65 (3H, s, $CH_3$), 1.56 (3H, s, $CH_3$), 1.45-1.28 (2H, m, 2 × ring CH), 1.13 (3H, s, $CH_3$) and 1.12 (3H, s, $CH_3$); $\delta_c$ (125 MHz, $CDCl_3$) 176.9, 176.0 (CO), 134.1, 119.9 (CH=CH), 52.1 (NH*C*HCO), 42.5 ($CH_2CMe_2$), 42.1 ($CH_2N$), 39.0, 31.5, 28.9, 28.0 ($CH_2$ lactam), 26.0, 25.0, 24.9, 17.9 ($CH_3$); m/z ($MH^+$ $C_{15}H_{27}N_2O_2$ requires 267.2073) 267.2063.

**Example 9: (*S*)-3-(2',2',5'-Trimethyl-hexanoyl)amino-caprolactam:**

(*S*)-3-(2',2',5'-trimethyl-hex-4'-enoyl)amino-caprolactam (400 mg) was dissolved in EtOAc (25 ml), palladium hydroxide-on-carbon (20%, *ca* 100 mg) was added, and the mixture was stirred at ambient temperature under an atmostsphere of hydrogen for 14 hours. The reaction was then filtered through a Celite® pad and the solvent was removed

*in vacuo* to give (*S*)-3-(2',2',5'-yrimethyl-hexanoyl)amino-caprolactam as a waxy solid (400 mg, 98%); m.p. 73-74 °C; $[\alpha]_D^{20}$ (c = 1, $CHCl_3$) +27.8; $\nu_{max}/cm^{-1}$ 3249 (NH), 1654, 1638 (CO), 1502 (NH); $\delta_H$ (500 MHz, $CDCl_3$) 7.08 (1 H, d, *J* 5.0, CHN*H*), 6.75-6.55 (1H, br m, $CH_2NH$), 4.44 (1H, ddd, *J* 11, 5.5, 1.5, C*H*NH), 329-3.16 (2H, m, $CH_2NH$), 2.03-1.91 (2H, m, 2 × ring CH), 1.84-1.73 (2H, m, 2 × ring CH), 1.47-1.28 (5H, m, 2 × ring CH + $CH_2$ + C*H*$(CH_3)_2$), 1.13 (3H, s, $CH_3$), 1.12 (3H, s, $CH_3$), 1.08-1.02 (2H, m, $CH_2$), 0.82 (3H, s, $CH_3$), 0.80 (3H, a, $CH_3$); $\delta_c$ (125 MHz, $CDCl_3$) 177.1, 176.1 (CO), 52.1 (NH*C*HCO), 42.1 ($CH_2N$), 41.9 ($CH_2CMe_2$), 39.0, 33.7, 31.5, 28.9 ($CH_2$), 28.4 ($Me_2CH$), 27.9 ($CH_2$), 25.3, 25.2, 22.6, 22.5 ($CH_3$); m/z ($MH^+$ $C_{15}H_{29}N_2O_2$ requires 269.2229) 269.2219.

**Reference Example 16: (*S*)-3-(11'-bromo-undecanoyl)amino-caprolactam:**

(*S*)-3-amino-caprolactam hydrochloride (5 mmol) and $Na_2CO_3$ (15 mmol) in water (25 ml) were added to a solution of 11-bromo-undecanoyl chloride (5 mmol) in dichloromethane (25 ml) at ambient temperature and the reaction was stirred for 4 hours. The organic layer was then separated and the aqueous phase was extracted with additional dichloromethane (2 × 25 ml). The combined organic layers were dried over $Na_2CO_3$ and reduced in *vacuo.* The residue was purified by recrystallisation from EtOAc to give **(*S*)-3-(11'-bromo-undecanoyl)amimo-caprolactam** (1.49 g, 79%); m.p. (EtOAc) 73-74°C; $[\alpha]_D^{25}$ (c = 1, $CHCl_3$) +31.8; $\nu_{max}/cm^{-1}$ 3342, 3287 (NH), 1668, 1634 (CO), 1515 (NH); $\delta_H$ (500 MHz, $d_6$-DMSO) 7.76 (1H, t, *J* 6.5, $CH_2NH$), 7.67 (1H, d, *J* 7, CHN*H*), 4.38 (1H, dd, *J* 11, 7, C*H*NH), 3.51 (2H, t, *J* 6.5, $CH_2Br$), 3.15 (1H, ddd, *J* 15.5, 10.5, 5, C*H*HNH), 3.05 (1H, dt, *J* 14, 7, CH*H*NH), 2.17-2.06 (2H, m, $CH_2CONH$), 1.85 (1H, dt, *J* 14, 3, C-5 H), 1.82-1.68 (4H, m, C-4 H, C-6 H and $CH_2CH_2Br$), 1.62 (1H, qt, *J* 12, 3.5, C-5 H), 1.46 (2H, br qn *J* 6.5, $CH_2CH_2CONH$), 1.41-1.31 (3H, m, C-4 H and chain $CH_2$) and 1.31-1.13 (11H, m, $(CH_2)_5$ + C-6 H); $\delta_c$ (125 MHz, $d_6$-DMSO) 174.4 (CO-ring), 171.3 (CO-chain), 51.3 (NH*C*HCO), 40.7 ($NCH_2$), 35.3, 35.2, 32.4, 31.3, 29.0, 28.9 (×3), 28.7, 28.2, 27.8, 27.6 and 25.4 ($CH_2$); m/z ($MH^+$ $BrC_{17}H_{32}N_2O_2$ requires 375.1647) 375.1655.

**Reference Example 17: (*S*)-3-(11'-azido-undecanoyl)amino-caprolactam;**

Sodium azide (650 mg, 10 mmol) was added to (*S*)-3-(11-bromo-undecanoyl)amino-caprolactam (375 mg, 1 mmol) in DMF (2 ml) and the mixture was heated at 60 °C for 14 hours. The solvent was then removed *in vacuo* and the residue was partitioned between water (20 ml) and EtOAc (3 × 20 ml). The combined organic layers were washed with 1 M $HCl_{aq}$ (2 × 20 ml) and then dried over $Na_2CO_3$ and reduced *in vacuo*. The residue was purified by recrystallisation from EtOAc to give ***(S)*-3-(11'-azido-undecanoyl)amino-caprolactam** (221 mg, 66%); m.p. (EtOAc) 71-72 °C; $[\alpha]_D^{25}$ (c=1, $CHCl_3$) +34.7; $\nu_{max}/cm^{-1}$ 3344, 3289 (NH), 2101 ($N_3$) 1668, 1631 (CO), 1516 (NH); $\delta_H$ (500 MHz, $d_6$-DMSO) 7.77 (1H, t, *J*6, $CH_2NH$), 7.67 (1H, d, *J*7, CHNH), 4.38 (1H, dd, *J* 11, 7, CHNH), 3.30 (2H, t, *J*7, $CH_2N_3$), 3.15 (1H, ddd, *J* 15.5, 10.5, 5, CHHNH), 3.05 (1H, dt, *J* 14, 5.5, CH*H*NH), 2.17-2.07 (2H, m, $CH_2CONH$), 1.85 (1H, dt, *J* 14, 3.5, C-5 H), 1.82-1.68 (2H, m, C-4 H, C-6 H), 1.62 (1H, qt, *J* 13, 3.5, C-5 H), 1.51 (4H, m, $CH_2CH_2CONH$ and $CH_2CH_2N_3$), 1.36 (1H, qd, *J* 13, 3, C-4 H), and 1.33-1.13 (13H, m, $(CH_2)_6$ + C-6 H); $\delta_C$ (125 MHz, $d_6$-DMSO) 174.4 (CO-ring), 171.3 (CO-chain), 51.3 (NHCHCO), 50.7 ($CH_2N_3$), 40.7 ($NCH_2$), 35.3, 31.3, 29.0 (×2), 28.9, 28.7, 28.6, 28.3, 27.8, 26.2 and 25.4 ($CH_2$); m/z ($MNa^+$ $C_{17}H_{31}N_5O_2Na$ requires 360.2375) 360.2360.

**Reference Example 18: (S) Sodium 3-(undecanoyl)amino-caprolactam 11'-sulfonate tetrahydrate:**

sodium sulfite (630 mg, 5 mmol) in water (3 ml) was added to (*S*)-3-(11-bromo-undecanoyl)amino-caprolactam (375 mg, 1 mmol) in ethanol (2 ml) and the mixture was heated at reflux for 14 hours. The cooled reaction mixture was then added to ethanol (25 ml) and the reaction was filtered. The solvent was then removed in *vacuo* to give **(*S*) sodium 3-(undecanoyl)amino-caprolactam 11'-sulfonate tetrahydrate** (456 mg, 97%); m.p. (EtOAc) 208-210 °C; $[\alpha]_D^{25}$(c = 1, $H_2O$) -15.5; $\nu_{max}/cm^{-1}$ 3430, 3344, 3289 (NH + $H_2O$), 1667, 1643 (CO), 1530 (NH) 1195, 1183 ($SO_3$, asymm.), 1064 ($SO_3$, symm.); $\delta_H$ (500 MHz, $d_6$-DMSO) 7.76 (1H, t, *J6*, $CH_2NH$), 7.70 (1H, d, *J7*, CHN*H*), 4.35 (1H, dd, *J* 10, 7.5, C*H*NH), 3.42 (8H, s 4 × $H_2O$) 3.17-3.00 (2H, m, $CH_2NH$), 2.47-2.38 (2H, m, $CH_2SO_3$), 2.17-2.05 (2H, m, $CH_2CONH$), 1.82 (1H, br s, *J* 13.5, C-5 H), 1.75-1.66 (2H, m, C-4 H, C-6 H), 1.65-1.50 (3H, m, C-5 H + chain $CH_2$), 1.47-1.40 (2H, m, chain $CH_2$) 1.35 (1H, qd, *J* 13, 3, C-4 H), and 1.30-1.11 (13H, m, $(CH_2)_6$ + C-6 H); $\delta_C$ (125 MHz, $d_6$-DMSO) 174.5 (CO-ring), 171.5 (CO-chain), 51.6 ($CH_2SO_3$), 51.4 (NHCHCO), 40.8 ($NCH_2$), 35.3, 31.3, 29.1 (×3), 29.0 (×2), 28.8, 28.6, 27.8, 25.5 and 25.1 ($CH_2$); m/z ($MNa^+$ $C_{17}H_{31}N_2O_5SNa_2$ requires 421.1749) 421.1748.

**Example 10: (S)-3-(Decanesulfonyl)amino-caprolactam:**

(*S*)-3-amino-caprolactam hydrochloride (3 mmol) and $Na_2CO_3$ (9 mmol) in water (20 ml) were added to a solution of decanesulfonylchloride (3 mmol) in dichloromethane (20 ml) at ambient temperature and the reaction was stirred for 10 hours. The organic layer was then separated and the aqueous phase was extracted with additional dichloromethane (2 × 25 ml). The combined organic layers were dried over $Na_2CO_3$ and reduced *in vacuo.* The residue was purified by recrystallisation from EtOAc / hexanes to give (S)-3-(decanesulfonyl)amino-caprolactam (481 mg, 48%); m.p. 98-99 °C; $[\alpha]_D^{25}$ (c =1, MeOH) +22.7; $v_{max}/cm^{-1}$ 3365, 3248 (NH), 1657 (CO), 1324, 1142 ($SO_2N$); $\delta_H$ (500 MHz, $CDCl_3$) 6.35-6.18 (1H, m, $CH_2NH$), 5.71 (1H, d, *J*6, CHNH), 4.11 (1H, ddd, *J* 11.5, 6, 2, CHNH), 3.31-3.18 (2H, m, C*H*$_2$NH), 2.98-2.92 (2H, m, $CH_2SO_2$, 2.09 (1H, br d, *J* 14, ring CH), 2.06-1.97 (1H, m, ring CH), 1.88-1.59 (5H, m, C*H*$_2$$CH_2SO_2$ +3 ring CH), 1.43-1.33 (3H, m, chain $CH_2$ + ring CH), 1.32-1.18 (12H, m, $CH_3(CH_2)_6$) and 0.86 (3H, m, $CH_3$); $\delta_C$ (125 MHz, $CDCl_3$) 174.8 (CO) 55.5 (NHCHCO), 53.5 ($CH_2SO_2$), 40.7 ($NCH_2$), 33.9, 31.8, 29.4, 29.3, 29,2, 29.1 , 28.6, 28.3, 27.9, 23.5, 22.6 ($CH_2$), and 14.1 ($CH_3$); m/z ($MNa^+$ $C_{16}H_{32}N_2O_3SNa$ requires 355.2031) 355.2054; anal ($C_{16}H_{32}N_2O_3S$ requires C, 57.8, H, 9.7, N, 8.4) C, 57.8, H, 9.7, N, 8.3.

**Example 11: (S)-3-(Dodecanesulfonyl)amino-caprolactam:**

(*S*)-3-amino-caprolactam hydrochloride (2 mmol) and $Na_2CO_3$ (6 mmol) in water (20 ml) were added to a solution of dodecanesulfonylchloride (2 mmol) in dichloromethane (20 ml) at ambient temperature and the reaction was stirred for 10 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (hexanes:EtOAc 3:1to 100% EtOAc) and then by recrystallisation from heptane to give **(*S*)-3-(dodecanesulfonyl)amino-caprolactam** (302 mg, 42%); m.p. 100-101 °C; $[\alpha]_D^{23}$ (c = 1, MeOH) +22.4; $v_{max}/cm^{-1}$ 3366,3247 (NH), 1657 (CO), 1324, 1143 ($SO_2N$); $\delta_H$ (500 MHz, $CDCl_3$) 6.66 (1H, t, *J*6, CH$_2$N*H*), 5.78 (1H, d, *J*6, CHN*H*), 4.10 (1H, ddd, *J*11, 6, 2, CHNH), 3.29-3.17 (2H, m, C*H*$_2$NH), 2.97-2.90 (2H, m, $CH_2SO_2$), 212-2.03 (1 H, m, ring CH), 2.03-1.96 (1H, m, ring CH), 1.88-1.59 (5H, m, C*H*$_2$$CH_2SO_2$ + 3 ring CH), 1.43-1.32 (3H, m, ring CH + chain $CH_2$), 1.32-1.18 (16H, m) and 0.85 (3H, m, $CH_3$); $\delta_C$ (125 MHz, $CDCl_3$) 175.0 (CO) 55.5 (NHCHCO), 53.5 ($CH_2SO_2$), 42.1 ($NCH_2$); 33.8, 31.8, 29.6 (×2), 29.5, 29.3 (×2), 29.1, 28.6, 28.3, 27.9, 23.5, 22.6 ($CH_2$), and 14.1 ($CH_3$); m/z ($MNa^+$ $C_{18}H_{36}N_2O_3SNa$ requires 383.2339) 383.2351; anal ($C_{18}H_{36}N_2O_3S$ requires C, 60.0, H, 10.1, N, 7.8) C, 59.9, H, 10.2, N, 7.7.

**Example 12: (S)-3-(Tetradecanesulfonyl)amino-caprolactam:**

(*S*)-3-amino-caprolactam hydrochloride (2 mmol) and $Na_2CO_3$ (6 mmol) in water (20 ml) were added to a solution of tetradecanesulfonylchloride (2 mmol) in dichloromethane (20 ml) at ambient temperature and the reaction was stirred for 10 hours. The organic layer was then separated and the aqueous phase was extracted with additional dichloromethane (2 × 25 ml). The combined organic layers were dried over $Na_2CO_3$ and reduced *in vacuo.* The residue was purified by silica column chromatography (hexanes:EtOAc 3:1 to 100% EtOAc) and then by recrystallisation from heptane to give **(*S*)-3-(tetradecanesulfonyl)amino-caprolactam** (373 mg, 48%); m.p. 100-101 °C; $[\alpha]_D^{21}$ (c = 1, $CHCl_3$) +14.4; $v_{max}/cm^{-1}$ 3361, 3250 (NH), 1658 (CO), 1324, 1140 ($SO_2N$); $\delta_H$ (500 MHz, $CDCl_3$) 6.64 (1H, t, *J*6, $CH_2NH$), 5.74 (1H, d, *J*6, CHNH), 4.11 (1H, ddd, *J*11.5, 6, 2, CHNH), 3.30-3.17 (2H, m, C*H*$_2$NH), 2.97-2.92 (2H, m, $CH_2SO_2$), 2.12-2.05 (1H, m, ring CH), 2.05-1.96 (1H, m, ring CH), 1.87-1.59 (5H, m, C*H*$_2$$CH_2SO_2$ + 3 ring CH), 1.42-1.32 (3H, m, ring CH + chain $CH_2$), 1.32-1.18 (20H, m, chain $CH_2$) and 0.86 (3H, m, $CH_3$); $\delta_C$ (125 MHz, $CDCl_3$) 174.9 (CO) 55.5 (NHCHCO), 53.4 ($CH_2SO_2$), 422 ($NCH_2$), 33.8, 31.9, 29.6 (×4), 29.5, 29.3 (×2), 29.1, 28.6, 28.3, 27.9, 23.5, 22.7 ($CH_2$), and 14.1 ($CH_3$); m/z ($MNa^+$ $C_{20}H_{40}N_2O_3SNa$ requires 411.2652) 411.2655; anal ($C_{20}H_{40}N_2O_3S$ requires C, 61.8, H, 10.4, N, 7.2) C, 61.9, H, 10.5, N, 7.2.

**Example 13: (S)-3-(Hexadecanesulfonyl)amino-caprolactam:**

(*S*)-3-amino-caprolactam hydrochloride (2 mmol) and $Na_2CO_3$ (6 mmol) in water (20 ml) were added to a solution of hexadecanesulfonylchloride (2 mmol) in dichloromethane (20 ml) at ambient temperature and the reaction

was stirred for 10 hours. The organic layer was then separated and the aqueous phase was extracted with additional dichloromethane (2 × 25 ml). The combined organic layers were dried over $Na_2CO_3$ and reduced *in vacuo*. The residue was purified by silica column chromatography (hexanes:EtOAc 3:1to 100% EtOAc) and then by recrystallisation from heptane to give **(*S*)-3-(hexadecanesulfonyl)amino-caprolactam** (553 mg, 66%); m.p. 100-101 °C; $[\alpha]^{25}_{D}$ (c = 1, $CHCl_3$) +14.1; $\nu_{max}/cm^{-1}$ 3356, 3249 (NH), 1659 (CO), 1323, 1140 ($SO_2N$); $\delta_H$ (500 MHz, $CDCl_3$) 6.55 (1H, t, *J*6, $CH_2NH$), 5.76 (1H*, d, J 6,* CHNR), 4.11 (1H, ddd, *J* 11.5, 6, 2, CHNH), 3.30-3.17 (2H, m, $CH_2NH$), 2.94 (2H, t, *J*8, $CH_2SO_2$), 2.12-2.04 (1H, m, ring CH), 2.04-1.97 (1H, m, ring CH), 1.87-1.58 (5H, m, $CH_2CH_2SO_2$ + 3 ring CH), 1.42-1.32 (3H, m, ring CH + chain $CH_2$), 1.32-1.18 (24H, m, chain $CH_2$) and 0.86 (3H, m, $CH_3$); $\delta_C$ (125 MHz, $CDCl_3$) 174.9 (CO) 55.5 (NHCHCO), 53.5 $(CH_2SO_2)_6$ 42.1 ($NCH_2$), 33.8, 31.9, 29.7 (×2), 29.6 (×4), 29.5, 29.3 (×2), 29.1, 28.6, 28.3, 27.9, 23.5, 22.7 ($CH_2$), and 14.1 ($CH_3$); m/z ($MNa^+$ $C_{20}H_{40}N_2O_3SNa$ requires 439.2965) 439.2980; anal ($C_{22}H_{44}N_2O_3S$ requires C, 63.4, H, 10.6, N, 6.7) C, 63.1, H, 10.6, N, 6.6.

**<u>Example 14:</u> (S)-3-(Octadecanesulfonyl)amino-caprolactam:**

(S)-3-amino-caprolactam hydrochloride (2 mmol) and $Na_2CO_3$ (6 mmol) in water (20 ml) were added to a solution of octadecanesulfonylchloride (2 mmol) in dichloromethane (20 ml) at ambient temperature and the reaction was stirred for 10 hours. The organic layer was then separated and the aqueous phase was extracted with additional dichloromethane (2 × 25 ml). The combined organic layers were dried over $Na_2CO_3$ and reduced *in vacuo*. The residue was purified by silica column chromatography (hexanes:EtOAc 3:1to 100% EtOAc) and then by recrystallisation from heptane to give **(*S*)-3-(octadecanesulfonyl)amino-caprolactam** (545 mg, 61%); m.p. 99-100 °C; $\nu_{max}/cm^{-1}$ 3356, 3249 (NH), 1659 (CO), 1323, 1140 ($SO_2N$); $\delta_H$ (500 MHz, $CDCl_3$) 6.15 (1H, t, *J*6, $CH_2NH$), 5.69 (1H, d, *J*6, CHNH), 4.12 (1H, ddd, *J* 11.5, 6, 2, C*H*NH), 3.30-3.18 (2H, m, $CH_2NH$), 2.97-2.92 (2H, m, $CH_2SO_2$), 2.12-2.07 (1H, m, ring CH), 2.06-1.97 (1H, m, ring CH), 1.87-1.56 (5H, m, $CH_2CH_2SO_2$ + 3 ring CH), 1.42-1.32 (3H, m, ring CH + chain $CH_2$), 1.32-1.18 (28H, m, chain $CH_2$) and 0.86 (3H, m, $CH_3$); m/z ($MNa^+$ $C_{24}H_{48}N_2O_3SNa$ requires 467.3277852) 467.330047.

**<u>Reference Example 19:</u>** (S)-aminocaprolactam-Glycine-(L)-N(Boc)- Tryptophan:

This tripeptide was made on a solid-phase automated peptide synthesiser using (S)-aminocaprolactam for the final peptide coupling step. Mr(Calc) = 471.5110. Observed Mr by mass spectrometry 471.6. Purity (%TIC in molecular ion peak) = 90%

**<u>Reference Example 20:</u> (S)-aminocaprolactam-(L)-valine-(L)-Decaminotryptophan**

This tripeptide was made on a solid-phase automated peptide synthesiser using (S)-aminocaprolactam for the final peptide coupling step. Mr(Calc) = 398.4600. Observed Mr by mass spectrometry 398.3. Purity (%TIC in molecular ion peak) = 96%

**<u>Reference Examples 21-24 of Intermediate Compounds useful in the synthesis of compounds of the invention:</u>**

**<u>Reference Example 21:</u>**

**Intermediate**

**(*E*)-Methyl 2,2-dimethyl-dodec-4-enoate:**

butyllithium (3.8 M, 10 mmol) was added to a solution of diisopropylamine (1.42 ml, 10 mmol) in dry THF at -78 °C under $N_2$. The reaction was strirred at -78 °C for 20 minutes and then methyl isobutyrate (1.15 ml, 10 mmol) was added. The reaction was stirred at -78 °C for 1 hour, and then (*E*)-dec-2-enyl bromide (2.19g, 10 mmol) was added and the reaction was allowed to warm to ambient temperature over 14 hours. The reaction solvent was then removed *in vacuo*, and the residue was partitioned between pH 2 aqueous buffer (0.5 M $NaHSO_4$ / 0.5 M $Na_2SO_4$) (100 ml) and hexane (3 × 100 ml). The combined organic layers were dried over $Na_2SO_4$ and the hexane solvent removed *in vacuo* to give crude (*E*)-methyl 2,2-dimethyl-dodec-4enoate (>90% pure) (2.27 g) as a colourless oil; $\nu_{max}/cm^{-1}$ 1734 (CO); $\delta_H$ (400 MHz, $CDCl_3$) 5.42 (1H, br dt, *J 15,* 6.5, CH=CH), 5.30 (1H, dtt, *J* 15. 7, 1, CH=C*H*), 3.64 (3H, s, $OCH_3$), 2.18 (2H, dd, *J* 7, 1, $CH_2CMe_2$), 1.96 (2H, br q, *J* 6.5, $CH_2CH_2CH$=CH), 1.35, 1.20 (10H, m, $(CH_2)_5CH_3$), 1.14 (6H, s, $C(CH_3)_2$), 0.87 (3H, t, *J* 6.5, $CH_2CH_3$); $\delta_C$ (125 MHz, $CDCl_3$) 178.2 (CO), 134,1, 125.2 (HC=CH), 51.5 ($OCH_3$), 43.6 ($CH_2$), 42,6

($Me_2CCO$), 32.6, 31.8, 29.5, 29.1, 29.0 ($CH_2$), 24.7 ($C(CH_3) \times 2$), 22.6 ($CH_2$), 14.1 ($CH_2CH_3$); m/z ($MH^+$ $C_{15}H_{29}N_2O_2$ requires 241.2168) 241.2169.

**Reference Example 22:**

**Intermediate**

**(*E*)-2,2-Dimethyl-dodec-4-enoyl chloride:**

the entire product from the above reaction was then dissolved in ethanol (50 ml) and added to a solution of NaOH (2.0 g, 50 mmol) in water (25 ml). The mixture was heated at reflux for 6 hours, allowed to cool and the solvents were then removed *in vacuo.* The residue was partitioned between pH 2 aqueous buffer (0.5 M $NaHSO_4$ / 0.5 M $Na_2SO_4$) (100 ml) and diethyl ether (3 × 100 ml). The combined organic layers were dried over $Na_2SO_4$ and the ether solvent removed in *vacuo* to give crude (E)-2,2-dimethyl-dodec-4-enoic acid (>90% pure) as a colourless oil; $\delta_H$ (400 MHz, $CDCl_3$) 5.46 (1 H, br dt, *J* 15, 6.5, *CH*=CH)*, 5.35* (1H, dtt, *J* 15, 7,1, CH=C*H*), 2.22 (2H, dd, *J*7, 1, $CH_2CMe_2$), 1.98 (2H, br q, *J* 6.5, $CH_2CH_2CH$=CH), 1.37-1.21 (10H, m, $(CH_2)_5CH_3$), 1.17 (6H, s, $C(CH_3)_2$), 0.87 (3H, t,*J*6.5, $CH_2CH_3$). The crude acid was dissolved in dichloromethane (50 ml) and oxalyl chloride (3 ml) was added along with a drop of DMF. The reaction was stirred for 1 hour and the solvent was removed *in vacuo* to give crude (*E*)-2,2-dimethyl-dodec-4-enoyl chloride which was all used without purification in the next step.

**Reference Example 23:**

**Intermediate**

**Methyl 2,2,5-trimethyl-hex-4-enoate:**

butyllithium (2.9 M, 50 mmol) was added to a solution of diisopropylamine (7.2 ml, 50 mmol) in dry THF (200 ml) at -78°C under $N_2$. The reaction was stirred at -78 °C for 20 minutes and then methyl isobutyrate (5.7 ml, 50 mmol) was added. The reaction was stirred at -78 °C for 1 hour, and then 3-methyl-but-2-enyl bromide (5.8 ml, 50 mmol) was added and the reaction was allowed to warm to ambient temperature over 14 hours. The reaction solvent was then removed *in vacuo,* and the residue was partitioned between pH 2 aqueous buffer (0.5 M $NaHSO_4$ / 0.5 M $Na_2SO_4$) and hexane (3 × 250 ml). The combined organic layers were dried over $Na_2SO_4$ and the hexane solvent removed in vacuo to give methyl 2,2,5-trimethyl-hex-4-enoate as a colourless oil (6.93 g 81%); $v_{max}/cm^{-1}$ 1732 (CO); $\delta_H$ (400 MHz, $CDCl_3$) 5.04 (1H, tsept, *J*7.5, 1.5, CH=C), 3.63 (3H, s, $OCH_3$), 2.20 (2H, d, *J*7.5, $CHCH_2$), 1.68 (3H, br s, CH=C*Me*Me), 1.58 (3H, br s, CH=CMe*Me*), 1.14 (6H, s, $(CH_3)_2CO$); $\delta_C$ (125 MHz, $CDCl_3$) 178.4 (CO), 134.1 ($Me_2C$=CH), 119.8 ($Me_2C$=*C*H), 51.6 ($OCH_3$), 42.8 ($Me_2CCO$), 38.7 ($CH_2$), 25.9, 24.7 (x 2), 17.8 ($CCH_3$); m/z ($MH^+$ $C_{10}H_{19}O_2$ requires 171.1385) 171.1388.

**Reference Example 24:**

**Intermediate**

**2,2,5-Trimethyl-hex-4-enoyl chloride:**

methyl 2,2,5-trimethyl-hex-4-enoate (2.74 g, 16 mmol) was dissolved in ethanol (50 ml) and added to a solution of NaOH (3.0 g, 75 mmol) in water (35 ml). The mixture was heated at reflux for 6 hours, allowed to cool and the solvents were then removed *in vacuo.* The residue was partitioned between pH 2 aqueous buffer (0.5 M $NaHSO_4$ / 0.5 M $Na_2SO_4$) and diethyl ether (3 x 150 ml). The combined organic layers were dried over $Na_2SO_4$ and the ether solvent removed *in vacuo* to give crude 2,2,5-trimethyl-hex-4-enoic acid (>95% pure) as a colourless oil; $\delta_H$ (400 MHz, $CDCl_3$) 5.12 (1H, tsept, *J* 7.5, 1.5, CH=C), 2.25 (2H, d, *J* 7.5, $CHCH_2$), 1.71 **(3H,** br s, CH=C*Me*Me), 1.60 (3H, br s, CH=CMe*Me*), 1.18 (6H, s, $(CH_3)_2CO$). The crude acid was dissolved in dichloromethane (50 ml) and oxalyl chloride (3 ml) was added along with a drop of DMF. The reaction was stirred for 1 hour and the solvent was removed *in vacuo* to give crude 2,2,5-trimethyl-hex-4-enoyl chloride which was all used without purification in the next step.

**Reference Example 25:**

This compound has two head groups on either side of a 2,2,6,6 tetramethyl heptanoic acid. It is in effect a dimer of the corresponding 2,2-dimethyl compound of the invention:

***(S,S) N,N'*-bis-(2'-oxo-azepan-3'-yl) 2,2,6,6-tetramethylheptadiamide:** (*S,S*)-3-amino-caprolactam hydro-pyrrolidine-5-carboaylate (2 mmol) and $Na_2CO_3$ (6 mmol) in water (25 ml) were added to a solution of 2,2,6,6-tetramethylheptandioyl dichloride (1 mmol) in dichloromethane (25 ml) at ambient temperature and the reaction was stirred for 2 hours. The organic layer was then separated and the aqueous phase was extracted with additional dichloromethane (2 x 25 ml). The combined organic layers were dried over $Na_2CO_3$ and reduced in *vacuo.* The residue was purified by recrystallisation from EtOAc to give **(*S,S*)-dimer** (199 mg, 46%); m.p. 234-236 °C; $[\alpha]_D^{25}$ (c =1, $CHCl_3$) +29.4; $v_{max}$/$cm^{-1}$ 3379, 3255 (NH), 1683, 1637 (CO), 1507, 1497 (NH); $\delta_H$ (500 MHz, $CDCl_3$) 7.07 (2H, d, *J* 5.5, CHN*H*), 6.42 (2H, br s, $CH_2$N*H*), 4.44 (2H, ddd, *J* 11, 5.5, 1.5, C*H*NH), 3.31-3.17 (4H, m, $CH_2$NH), 2.04-1.94 (4H, m, ring CH), 1.86-1.73 (4H, m, ring CH), 1.51-1.31 (8H, br m, 2 x ring CH + $CH_2CMe_2$) and 1.12 (14H, m, chain $CH_2CH_2CH_2$ + $CMe_2$); $\delta_c$ (125 MHz, $CDCl_3$) 176.9, 175.9 (CO), 52.1 (NHCH), 42.0 ($CMe_2$), 42.1, 41.5, 31.5, 28.9, 28.0 ($CH_2$), 25.3, 25.1 ($CH_3$) and 20.0 ($CH_2$); m/z ($M^+$ $C_{23}H_{40}N_4O_4$ requires 436.30496) 436.30437.

**<u>Example 15:</u> (*S*)-3-(1',1'-dimethylundecanesulfonyl)amino-caprolactam**

This compound is the sulfonamide analogue of Example 1.

**<u>Reference Example 26:</u> (*S*)-3-(2'-Propylpentanoyl)amino-caprolactam:**

(*S,S*)-3-amino-caprolactam hydro-pyrrolidine-5-carboxylate (5 mmol) and $Na_2CO_3$ (15 mmol) in water (13 ml) were added to a solution of 2-propylpentanoyl chloride (5 mmol) in dichloromethane (15 ml) at ambient temperature and the reaction was stirred for 12 hours. The organic layer was then separated and the aqueous phase was extracted with additional dichloromethane (2 × 25 ml). The combined organic layers were dried over $Na_2SO_4$ and reduced *in vacuo.* The residue was recrystallised from hexane to give (S)-3-(2'-propylpentanoyl)amino-caprolactam (1.02 g, 80%); m.p. (hexanes) 114-118 °C; $[\alpha]_D^{25}$(c = 1, $CHCl_3$) +29.4; $v_{max}$/$cm^{-1}$ 3303 (NH), 1686, 1633 (CO), 1537 (NH); $\delta_H$ (500 MHz, $CDCl_3$) 6.88 (1H, d, *J* 5.5, CHN*H*), 6.52 (1H, br s, $CH_2$N*H*), 4.52 (1 H, ddd, *J* 11, 6, 1.5, C*H*NH), 3.30-3.16 (2H, m, $CH_2$NH), 2.13-2.02 (2H, m, $(CH_2)_2$C*H*CO and lactam ring CH), 2.02-1.92 (1H, m, lactam ring CH), 1.86-1.74 (2H, m, lactam ring CH $x_2$), 1.57-1.50 (2H, m, sidechain $CH_2$), 1.42 (1H, br qd, *J* 13.5. 3.5, lactam ring CH), 1.38-1.29 (2H, m, lactam ring CH + side chain $CH_2$), 1.29-1.19 (4H, m, sidechain CH x4), 0.85 (3H, t, *J* 7.5, $CH_3$) and 0.84 (3H, t, *J* 7.5, $CH_3$); $\delta_c$ (125 MHz, $CDCl_3$) 175.8, 175.2 (CO), 51.9 (NHCHCO), 47.2 (CH), 42.1, 35.3, 35.1, 31.7, 28.9, 27.9, 20.7 (x2) ($CH_2$) and 14.1 (x2) ($CH_3$); m/z ($MH^+$ $C_{14}H_{27}N_2O_2$ requires 255.2073) 255.2083.

**<u>Reference Example 27(a):</u> (3S,2'R) and <u>Reference Example 27(b)</u>: (3*S*,2'*S*)-3-(2'-Ethylhexanoyl)amino-caprolaetam:**

(*S,S*)-3-amino-caprolactam hydro-pyrrohdine-5-carboxylate (5 mmol) and $Na_2CO_3$, (15 mmol) in water (15 ml) were added to a solution of (+/-) 2-ethylhexanoyl chloride (5 mmol) in dichloromethane (15 ml) at ambient temperature and the reaction was stirred for 12 hours. The organic layer was then separated and the aqueous phase was extracted with additional dichloromethane (2 x 25 ml). The combined organic layers were dried over $Na_2SO_4$ and reduced in *vacuo.* The residue was recrystallised from hexane to give a mixture of (3*S*,2'*R*) and (3*S*,2'*S*)-3-(2'-ethylhexanoyl)amino-caprolactam (328 mg, 26%); $v_{max}$/$cm^{-1}$ 3306 (NH), 1686, 1633 (CO), 1537 (NH); $\delta_H$ (500 MHz, $CDCl_3$) 6.89 (2H, d, *J* 5, CHN*H*, both isomers), 6.53 (2H, br s, $CH_2$N*H*, both isomers), 4.52 (2H, ddd, *J* 11.6,1.5, C*H*NH, both isomers), 3.30-3.16 (4H, m, $CH_2$NH, both isomers), 2.06 (2H, br d, *J* 13.5, lactam CH x2, both isomers), 2.02-1.92 (4H, m, $(CH_2)_2$C*H*CO $x_2$ and lactam ring *C*H x2, both isomers), 1.86-1.74 (4H, m, lactam ring CH ×4, both isomers), 1.63-1.50 (4H, m, sidechain $CH_2$), 1.50-1.30 (8H, m, lactam ring CH ×4 + sidechain $CH_2$ x4, both isomers), 1.30-1.14 (8H, m, side chain $CH_2$ ×8, both isomers), 0.85 (3H, t, *J* 7.5, $CH_3$, one isomer) and 0.82 (3H, t, *J* 7.5, $CH_3$, one isomer); $\delta_c$ (125 MHz, $CDCl_3$) 175.8, 175.1 (CO), 52.0, 51.9 (NH*C*HCO), 49.3 (x2) (CH), 42.0 (x2), 32.5, 32.3, 31.7 (x2), 29.7 (x2), 28.8 (x2), 27.9 (x2), 26.1, 25.9, 22.7 (x2), 14.0, 13.9 ($CH_3$) and 12.0 (x2) ($CH_3$); m/z ($M^+$ $C_{14}H_{26}N_2O_2$ requires 254.1994) 254.1995.

**<u>Reference Example 28:</u> 3,3-Dimethyldodecanoic acid (Intermediate)**

CuI (2 mmol), trimethylsilyl chloride (24 mmol) and methyl 3,3-dimethylacrylate (20 mmol) in THF (25 mmol) was cooled to -15 °C, and a solution of nonylmagnesium bromide (24 mmol) in THF (80 ml) was added over one hour. The reaction was allowed to warm to room temperature overnight and it was then quenched by the addition of saturated

aqueous ammonium chloride. The THF was removed *in vacuo* and the residue was partitioned between hexanes and water. The organic layer was reduced *in vacuo* and the crude methyl 3,3-dimethyldodecanoate was dissolved in ethanol (50 ml). KOH (100 mmol) in water (10 ml) was added and the reaction was heated at reflux for 18 hours. The reaction was then allowed to cool, and the solvent was removed *in vacuo.* and the resudue was partitioned between hexane and water. The aqueous layer was then acidified to pH 2 with aqueous HCl. and extracted with diethyl ether. The ether layer was dried over $Na_2SO_4$ and the solution was then reduced in vacuo to give 3,3-dimethyldodecanoic acid as an oil (3.47 g, 76%); $v_{max}/cm^{-1}$ 1702 (CO); $\delta_H$ (500 MHz, $CDCl_3$) 11.12 (1H, br s, OH), 2.21 (2H, s, $CH_2CO$); 1.32-1.20 (16H, m, $(CH_2)_8$), 1.00 (6H, s, $C(CH_3)_2$) and 0.87 (3H, t, *J* 7, $CH_2CH_3$); $\delta_c$ (125 MHz, $CDCl_3$) 179.1 (CO),45.9, 42.3 ($CH_2$), 33.2 (*C*$(CH_3)_2$), 31.9, 30.3, 29.6 (×2), 29.3, 27.1 (×2) ($C(CH_3)_2$), 24.0, 22.6 ($CH_2$) and 14.1 ($CH_3$); m/z ($M^+$ $C_{14}H_{28}O_2$ requires 228.2089) 228.2082.

**__Reference Example 29:__ 3,3-Dimethyldodecanoyl chloride (Intermediate)**

3,3-dimethyldodecanoic acid (5 mmol) was dissolved in $CH_2Cl_2$ (20 ml) oxalyl chloride (1 ml) and dimethyl formamide (1 drop) was added. After 1 hour the reaction was reduced *in vacuo* to give crude 3,3-dimethyldodecanoyl chloride which was used directly in the synthesis of (*S*)-3-(3',3'-dimethyldodecanoyl)amino-caprolactam.

**__Reference Example 30:__ (*S*)-3-(3',3'-Dimethyldodecanoyl)amino-caprolactam:**

(*S,S*)-3-amino-caprolactam hydro-pyrrolidino-5-carboxylate **2** (5 mmol) and $Na_2CO_3$ (15 mmol) in water (15 ml) were added to a solution of 3,3-dimethyldodecanoyl chloride (5 mmol) in dichloromethane (15 ml) at ambient temperature and the reaction was stirred for 12 hours. The organic layer was then separated and the aqueous phase was extracted with additional dichloromethane (2 x 25 ml). The combined organic layers were dried over $Na_2SO_4$ and reduced *in vacuo.* The residue was recrystallised from hexane to give (*S*)-3-(3',3'-dimethyldodecanoyl)amino-caprolactam (1.14 g, 68%); m.p. (hexanes) 123-125 °C; $[\alpha]_D$ (c= 1, $CHCl_3$) +28.6; $v_{max}/cm^{-1}$ 3279 (NH), 1646 (CO), 1498 (NH); $\delta_H$ (500 MHz, $CDCl_3$) 6.81 (1H, d, *J* 5.5, CHN*H*), 6.59-6.42 (1H, br m, $CH_2NH$), 4.50 (1H, ddd, *J* 11, 6, 1.5, C*H*NH), 3.30-3.16 (2H, m, $CH_2NH$), 2.08-2.02 (3H, m, $CH_2CO$ + lactam ring CH), 2.00-1.90 (1H, m, lactam ring CH), 1.86-1.75 (2H, m, lactam ring CH ×2), 1.47-1.31 (2H, br m, lactam ring CH ×2), 1.30-1.17(16H, m, $(CH_2)_8$), 0.89 (6H, s, $C(CH_3)_2$) and 0.84 (3H, t, *J* 7, $CH_2CH_3$); $\delta_c$ (125 MHz, $CDCl_3$) 175.8, 170.9 (CO), 52.0 (NH*C*H), 48.4, 42.6, 41.1 ($CH_2$), 33.3 (*C*$Me_2$), 31.9, 31.7, 30.4, 29.7,29.6, 29,3, 28.9, 27.9 ($CH_2$), 27.3 (×2) ($CH_3$), 24.1, 22.6 ($CH_2$) and 14.1 ($CH_3$); m/z ($M^+$ $C_{20}H_{38}N_2O_2$ requires 338.2933) 338.2928.

**__Reference Example 31:__ (B)-Ethyl 2-methyldodec-2-enoate (Intermediate)**

Decanal (5 mmol) and (carbethoxyethylidene)triphenylphosphorane (10 mmol) were dissolved in $CH_2Cl_2$ (20 ml)and the reaction was stirred for 18 hours. The solvent was then removed *in vacuo* and the residue was filter through a plug of silica gel with the aid of 5% diethyl ether in hexanes. The collected eluent was reduced *in vacuo* to give (*E*)-ethyl 2-methyldodec-2-enoate as an oil (1.02 g, 88%); $v_{max}/cm^{-1}$ 1709 (CO), 1651 (C=C); $\delta_H$ (500 MHz, $CDCl_3$) 6.73 (1H, tq, *J* 7.5, 1.5, CH=C), 4.16 (2H, q, *J* 7, $OCH_2$), 2.13 (2H, br q, *J* 7.5, $CH_2CH$=C), 1.80 (3H, d, *J* 1.5, $CH_3C$=CH), 1.45-1.37 (2H, m, chain $CH_2$), 1.32-1.19 (15H, m, $(CH_2)_6$ + $OCH_2CH_3$) and 0.85 (3H, t, *J* 7, $(CH_2)_8CH_3$); $\delta_c$ (125 MHz, $CDCl_3$) 168.3 (CO), 142.4 (*C*H=C), 127.6 (CH=C), 60.3 ($OCH_2$), 31.8, 29.5, 29.4 (x2), 29.3, 28.6, 28.5, 22.6 ($CH_2$), 14.3, 14.1 and 12.3 ($CH_3$); m/z ($MH^+$ $C_{15}H_{29}O_2$ requires 241.2168) 241.2165.

**__Reference Example 32:__ (*E*)-2-Methyldodec-2-enoic acid (Intermediate)**

(*E*)-Ethyl 2-methyldodec-2-enoate (1.43 mmol) was dissolved in ethanol (10 ml), and KOH (10 mmol) in water (5 ml) was added. The reaction was heated at reflux for 18 hours and then cooled. The solvent was removed *in vacuo* and the residue partitioned between water and hexane. The aqueous layer was acidified with aqueous HCl, and was extracted with diethyl ether. The diethyl ether layer was dried over $Na_2SO_4$ and reduced *in vacuo* to give (*E*)-2-methyldodec-2-enoic acid as a solid (308 mg, >95%); m.p. 28-31 °C; $\delta_H$ (400 MHz, CDCl,) 6.91 (1H, tq, *J* 7.5, 1.5, CH=C), 2.18 (2H, br q, *J* 7.5, $CH_2CH$=C), 1.82 (3H, d, *J 1.5,* $CH_3C$=CH), 1.48-1.39 (2H, m, chain $CH_2$),1.36-1.19 (12H, m, $(CH_2)_6$) and 0.88 (3H, t, *J* 7, $(CH_2)_8CH_3$) (no OH peak observed).

**__Reference Example 33:__ (*E*)-2-Methyldodec-1-enoyl chloride (Intermediate)**

(*E*)-2-Methyldodec-2-enoic acid (1.43 mmol) was dissolved in $CH_2Cl_2$ (20 ml) oxalyl chloride (1 ml) and dimethyl

formamide (1 drop) was added. After 1 hour the reaction was reduced *in vacuo* to give crude (*E*)-2-methyldodec-2-enoyl chloride which was used directly in the synthesis of (*S*)-(*E*)-3-(2'-methyldodec-2'-enoyl)amino-caprolactam.

**Reference Example 34: (*S*)-(*E*)-3-(2'-Methyldodec-2'-enoyl)amino-caprolactam:**

(*S,S*)-3-amino-caprolactam hydro-pyrrolidine-5-carboxylate **2** (2 mmol) and $Na_2CO_3$ (6 mmol) in water (15 ml) were added to a solution of (*E*)-2-methyldodec-2-enoyl chloride (1.43 mmol) in dichloromethane (15 ml) at ambient temperature and the reaction was stirred for 12 hours. The organic layer was then separated and the aqueous phase was extracted with additional dichloromethane (2 × 25 ml). The combined organic layers were dried over $Na_2SO_4$ and reduced *in vacuo.* The residue was recrystallised from hexane to give (*S*)-(*E*)-3-(2'-methyldodec-2'-enoyl)amino-caprolactam (297 mg, 65%); m.p. (hexanes) 99-100 °C; $\nu_{max}/cm^{-1}$ 3282 (NH), 1656,1622 (CO and C=C), 1497 (NH); $[\alpha]_D^{25}$ (c = 1, $CHCl_3$) +38.2; $\delta_H$ (500 MHz, $CDCl_3$) 7.15 (1H, d, *J* 5.5, N*H*CH), 6.48-6.35 (2H, m, N*H*$CH_2$ + CH=C), 4.54 (1H, ddd, *J* 11, 5.5, 1.5, NHC*H*), 3.33-3.17 (2H, m, C$H_2$NH), 2.14-2.05 (3H, m, C$H_2$CH=C + lactam ring CH), 2.02-1.93 (1H, m, lactam ring CH), 1.88-1.77 (5H, m, lactam ring CH ×2 + C$H_3$C=CH), 1.47-1.31 (4H, br m, lactam ring CH ×2 + chain $CH_2$), 1.31-1.17 (12H, m, $(CH_2)_6$) and 0.85 (3H, t, *J* 7, $CH_2CH_3$); $\delta_c$ (125 MHz, $CDCl_3$) 175.9, 168.2 (CO), 136.9 (*C*H=C), 130.2 (CH=*C*), 52.3 (NHCH), 42.2 ($NHCH_2$), 31.8, 31,6, 29.5, 29.4 (x2), 29.3, 28.9, 28.7, 28.3, 27.9, 22.6 ($CH_2$), 14.1 and 12.4 ($CH_3$).

**Reference Example 35(a): (3*S*,2'*R*) and**

**Reference Example 35(b): (3*S*,2'*S*)-3-(2'Methyldodecanoyl)amino-caprolactam:**

(*S*)-(*E*)-3-(2'-Methyldodec-2'-enoyl)amino-caprolactam (0.5 mmol) and $Pd(OH)_2$ (20% on carbon) were added to methanol (10 ml) and the mixture was stirred for 18 hours at ambient temperature under an atmosphere of hydrogen. The reaction was then fIltered, and the solvent removed *in vacuo* to give (3*S*,2'*R*) and (3*S*,2'*S*)-3-(2'-methyldodecanoyl) amino-caprolactam as a solid (160 mg, >95%); $\nu_{max}/cm^{-1}$ 3313 (NH), 1671, 1636 (CO), 1515 (NH); $\delta_H$ (500 MHz, $CDCl_3$) 6.91 (2H, d, *J* 5.5, CHN*H*, both isomers), 6.55 (2H, br s, $CH_2$N*H*, both isomers), 4.57-4.47 (2H, m, C*H*NH, both isomers), 3.34-3.18 (4H, m, C$H_2$NH, both isomers), 2.29-2.14 (2H, $CH_3$C*H*CO, both isomers), 2.07 (2H, br d, *J* 13.5, lactam ring CH, both isomers), 2.02-1.94 (2H, m, lactam ring CH, both isomers), 1.89-1.76 (4H, m, lactam ring CH ×2, both isomers), 1.67-1.57 (2H, m, chain CH, both isomers), 1.51-1.33 (6H, m, lactam ring CH ×2 + side chain $CH_2$, both isomers), 1.32-1.18 (32H, m, $(CH_2)_8$, both isomers), 1.13 (3H, d, *J* 7, CHC$H_3$, one isomer), 1.11 (3H, d, *J* 7, CHC$H_3$, one isomer) and 0.87 (6H, t, *J* 7.5, $CH_3$, both isomers); $\delta_c$ (125 MHz, $CDCl_3$) 175.9 (×2), 175.8 (×2) (CO, both isomers), 52.0, 51.9 (NCH), 42.1 (×2) ($NCH_2$, both isomers), 41.3, 41.2 ($CHCH_3$), 34.5, 34.1, 31.9 (x2), 31.8, 31.7, 29.6 (x6), 29.5 (x2), 29.3 (x2), 28.9 (x2), 28.0, 27.9, 27.4 (x2), 22.6 (x2) ($CH_2$) 17.8, 17.6 and 14.1 (×2) ($CH_3$); m/z ($MH^+$ $C_{19}H_{37}N_2O_2$ requires 325.2855) 325.2858.

**Reference Example 36: (4*S*,2'*S*,3'*R*)-4-Benzyl-3-(3'-hydroxy-2'-methyldecanoyl)-oxazolidin-2-one (Intermediate)**

This aldol reaction was performed according to published method (Crimmins, M.T; She, J.; Synlett, 2004, 1371-1374). (*S*)-4-Benzyl-3-propianyl-oxazolidin-2-one (5 mmol) (synthesised according to the method of Evans et al. Tetrahedron Lett., 1987, 28, 1123) was dissolved in $CH_2Cl_2$ (25 ml) and the solution was cooled to-20 °C under an atmosphere of dry nitrogen and $TiCl_4$ (5.25 mmol) was added. After 15 minutes, diisopropylethylamine (5.5 mmol) was added. After a further 40 minutes *N*-methylpyrrolidin-2-one (5.25 mmol) was added. After a further 10 minutes, decanal (5.5 mmol) was added and the reaction was stirred for 1 hour. Ammonium chloride solution was added and the reaction mixture was washed with pH 2 buffer (0.5 M $Na_2SO_4$ / 0.5 M $NaHSO_4$). The organic layer was dried over $Na_2SO_4$ and reduced in vacuo. The crude product was chromatographed on silica gel (10% to 33% ethyl acetate in hexane) to give (4S,2'S,3'R)-4-benzyl-3-(3*-hydroxy-2'-methyldecanoyl)-oxazolidin-2-one as an oil (1.34 g, 69%); $\nu_{max}/cm^{-1}$ 1778 ($NCO_2$), 1697 (CON); $\delta_H$ (500 MHz, $CDCl_3$) 7.35-7.30 (2H, m, meta-Ph), 7.29-7.24 (1H, m, para-Ph), 7.21-7.17 (2H, m, *ortho*-Ph), 4.69 (1H, ddt, *J* 9.5, 7.5, 3.5, CHN), 4.21 (1H, t, *J* 9, OC*H*H), 4.17 (1H, dd, *J* 9, 3, OCH*H*), 3.93 (1H, ddd, *J* 7, 4.5, 3, C*H*OH), 3.75 (1H, qd, *J* 7, 2.5, $CHCH_3$), 3.24 (1H, dd, *J* 13.5, 3.5, C*H*HPh), 2.87 (1H, br s, CHO*H*), 2.78 (1H, dd, *J* 13.5, 9.5, CH*H*Ph), 1.56-1.20 (19H, m, $(CH_2)_8$ + CHC$H_3$) and 0.86 (3H, t, *J* 7, $CH_2$C$H_5$); $\delta_C$ (125 MHz, $CDCl_3$) 177.6 (C*C*O), 153.0 (OCO), 135.0 (*ipso*-Ph), 129.4, 129.0 (*ortho*- + *meta*- Ph), 127.4 (*para*-Ph), 71.5 (CHOH), 66.1 ($OCH_2$), 55.1 (NCH), 42.1 (*C*$HCH_3$), 37.8, 33.8, 31.9, 29.6 (×3), 29.3, 26.0, 22.7 ($CH_2$), 14.1 and 103 ($CH_3$); m/z ($MH^+$ $C_{23}H_{36}NO_4$ requires 390.2644) 390.2641.

**Reference Example 37: (4*R*,2'*R*,3'*S*)-4-Benzyl-3-(3'-hydroxy-2-methyldecanoyl)-oxazolidin-2-one (Intermediate)**

(*R*)-4-Benzyl-3-propionyl-oxazolidin-2-one was converted into (4*R*,2'*R*,3'*S*)-4-benzyl-3-(3'-hydroxy-2'-methyldecanoyl)-oxazolidin-2-one according to the above procedure. NMR spectroscopic data is identical; m/z ($MH^+$ $C_{23}H_{36}NO_4$ requires 390.2644) 390.2638.

**Reference Example 38: (2*S*,3*R*)-3-Hydroxy-2-methyldecanoic acid (Intermediate)**

(4*S*,2'*S*,3'*R*)-4-Benzyl-3-(3'-hydroxyl-2'-methyldecanoyl)-oxazolidin-2-one (1.42 mmol) was dissolved in THF (10 ml). Water (2 ml), aqueous hydrogen peroxide (8M, 0.5 mmol) and $LiOH.H_2O$ (3 mmol) were added, and the reaction was stirred for 18 hours. $Na_2SO_3$ (10 mmol) was added and the reaction was extracted with ethyl acetate. The aqueous layer was then acidified with pH 2 buffer (0.5 M $Na_2So_4$ / 0.5 M $NaHSO_4$), and extracted with diethyl ether. The diethyl ether layer was dried over $Na_2SO_4$ and reduced in vacuo to give crude (2*S*,3*R*)-3-hydroxy-2-methyldecanoic acid; $\delta_H$ (400 MHz, $CDCl_3$) 3.96-3.89 (1H, m, C*H*OH), 2.59 (1H, dq, *J*7, 3, C*H*$CH_3$), 1.54-1.36 (2H, m, $CH_2$), 1.36-122 (14H, m, $(CH_2)_7$) and 1.20 (3H, d, *J* 7, CHC$H_3$). This material was used directly in the synthesis of (3*S*,2'*S*,3'*R*)-3-(3'-hydroxy-2'-methyldecanoyl)amino-caprolactam.

**Reference Example 39: (2*R*,3*S*)-3-Hydroxy-2-methyldecanoic acid (Intermediate)**

(2*R*,3*S*)-3-Hydroxy-methyldecanoic acid was prepared from (4*R*,2'*R*,3'*S*)-4-benzyl-3-(3'-hydroxy-2'-methyldecanoyl)-oxazolidin-2-one according to the above procedure.

**Reference Example 40: (*3*S,2'*S*,3'*R*)-3-(3'-Hydroxy-2'-methyldecanoyl)amino-caprolactam:**

(2*S*,3*R*)-3-Hydroxy-2-methyldecanoic acid (1.40 mmol) was dissolved in MeOH (10 ml), and (*S*)-3-amino-caprolactam hydrochloride (1.50 mmol) and triethylamine (2 mmol) were added. The reaction was cooled to 0 °C and 4-(4,6-dimethoxy[1,3,5]triazin-2-yl)-4-methyl-morpholinium chloride (1.40 mmol) was added. The reaction was stirred for 4 hours, and then the solvent was removed *in vacuo.* The residue was partitioned between ethyl acetate and water. The ethyl acetate layer was washed with dilute aqueous HCl and dilute aqueous NaOH, and then dried over $Na_2SO_4$. The solvent was removed *in vacuo* and the residue was recrystallised form ethyl acetate / hexane to give (3*S*,2'*S*,3'*R*)-3-(3'-hydroxy-2'-methyldecanoyl)amino-caprolactam as a solid (341 mg, 72%) m.p. (hexanes) 88-91 °C; $\nu_{max}/cm^{-1}$ 3313 (NH), 1628 (CO), 1480 (NH); $[\alpha]_D$ (c= 0.5, $CHCl_3$) +40.8; $\delta_H$ (500 MHx, $CDCl_3$) 7.08 (1H, d *J* 5.5, N*H*CH), 6.51 (1H, br s, N*H*$CH_2$), 4.57 (1H, ddd, *J*11, 6.5, 1, NCH), 3.83 (1H, dt, *J* 8, 4, C*H*OH), 3.36-3.21 (2H, m, $NCH_2$), 2.40 (1H, dq, *J* 7, 3, $CHCH_3$), 2.12-1.96 (2H, m, lactam CH $\times$2),1.90-1.76 (2H, m, lactam CH $\times$2), 1.55-1.34 (4H, m, lactam CH $\times$2 + chain $CH_2$), 1.34-1.21 (14H, m, $(CH_2)_7$), 1.17 (3H, d, *J* 7, CHC$H_3$) and 0.88 (3H, t, *J* 7, $CH_2$C$H_3$) (OH not observed); $\delta_C$ (125 MHz, $CDCl_3$) 175.8, 175.7 (CO), 72.1 (CHOH), 52.0 (NCH), 44.6 (*C*$HCH_3$), 42.1 ($NCH_2$), 33.4, 31.9, 1.3, 9.6 ($\times$2), 29.5, 29.3, 28.8, 27.9, 26.1, 22.7 ($CH_2$), 14.1 and 11.2 ($CH_3$); m/z ($MH^+$ $C_{19}H_{37}N_2O_3$ requires 341.2804) 341.2776.

**Reference Example 41: (3*S*,2'*R*,3'*S*')-3-(3'-Hydroxy-2'-methyldecanoyl)amino-caprolactam:**

(2*R*,3*S*)-3-Hydroxy-2-methyldecanoic acid (1.40 mmol), (*S*)-3-amino-caprolactam hydrochloride (1.50 mmol), triethylamine (2 mmol), and 4-(4,6-dimethoxy[1,3,5]triazin-2-yl)-4-methyl-morpholiniumn chloride (1.40 mmol) were reacted together, as above to produce (3*S*,2'*R*,3*S*)-3-(3'-hydrox)-2'-methyldecanoyl)amino-caprolactam which was recrystallised from ethyl acetate /hexane (86 mg, 18%); m.p. (hexanes) 118-121 °C; vmax/$cm^{-1}$ 3294 (NH), 1667, 1613 (CO), 1533 (NH); $[\alpha]_D$ (c = 0.5, $CHCl_3$) +14.8; $\delta_H$ (500 MHz, $CDCl_3$) 7.11 (1H, d, *J* 6, N*H*CH), 6.54 (1H, br s, N*H*$CH_2$), 4.53 (1H, ddd, *J* 11, 6.5, 1.5, NCH), 3.87-3.80 (1H, m, C*H*OH), 3.70 (1H, br s, OH), 3.34-3.20 (2H, m, $NCH_2$), 2.37 (1H, dq, *J* 7, 3, C*H*$CH_3$), 2.11-1.96 (2H, m, lactam CH $\times$2), 1.90-1.76 (2H, m, lactam CH $\times$2),1.55-1.21 (18H, m, lactam CH $\times_2$ + chain $(CH_2)_8$), 1.16 (3H, d, *J* 7, CHC$H_3$) and 0.88 (3H, t, *J* 7, $CH_2$C$H_3$); δc (125 MHz, $CDCl_3$) 175.9,175.7 (CO), 72.0 (CHOH), 52.1 (NCH), 44.8 (*C*$HCH_3$), 42.1 ($NCH_2$), 33.7, 31.9, 31.4, 29.6 ($\times$2), 29.5, 29.3, 28.8, 27.9, 26.0, 22.7 ($CH_2$),14.1 and 10.7 ($CH_3$); m/z ($MH^+$ $C_{19}H_{37}N_2O_3$ requires 341.2804) 341.2803.

**Reference Example 42:Methyl 2,2-dimethyl-3-hydroxy decanoate (intermediate)**

Butyllithium (2.5 M in hexanes, 50 mmol) was added to a solution of diisopropylamine (50 mmol) in dry THF (200 ml) at -78°C under an atmosphere of dry nitrogen. The reaction was stirred for 30 minutes, and then methyl isobutyrate (50 mmol) was added. After 45 minutes, decanal (50 mmol) was added and the reaction was allowed to warm to ambient temperature over 18 hours. After the addition of saturated aqueous ammonium chloride (10 ml), the reaction solvent was removed *in vacuo* and the residue was partitioned between hexanes and pH 2 buffer (0.5 M $Na_2SO_4$ / 0.5 M $NaHSO_4$). The organic layer was dried over $Na_2SO_4$ and the solvent was removed to give methyl 2,2-dimethyl-3-hydroxy decanoate as an oil (9.98g, 77%); $\delta_H$ (400 MHz, $CDCl_3$) 3.70 (3H, s, $OCH_3$), 3.69 (1H, dd, *J* 10, 2, C*H*OH), 1.68-1.20 (16H, m, $(CH_2)_8$), 1.19 (3H, s, $CCH_3$), 1.17 (3H, s, $CCH_3$) and 0.88 (3H, t, *J* 7, $CH_2CH_3$) (no OH observed).

**Reference Example 43: 2,2-Dimethyl-3-hydroxy decanoic acid (Intermediate)**

Methyl 2,2-dimethyl-3-hydroxy decanoate (20 mmol) was dissolved in EtOH (80 ml) and a solution of KOH (40 mmol) in water (20 ml) was added. The reaction was heated at reflux for 18 hours, and then the reaction was allowed to cooL The solvent was removed *in vacuo* and the residue was partitioned between water and diethyl ether. The aqueous layer was then acidified with pH 2 buffer (0.5 M $Na_2SO_4$ / 0.5 M $NaHSO_4$) and extracted with diethyl ether. The solution was dried over $Na_2SO_4$ and rediced in *vacuo* to give 2,2-dimethyl-3-hydroxydecanoic acid which solidified on standing; m.p. 39-41 °C; $\delta_H$ (400 MHz, $CDCl_3$) 3.64 (1H, dd, *J* 10, 2, CHOH), 1.67-1.12 (22H, m, $(CH_2)_8 + C(CH_3)_2$) and 0.88 (3H, t, *J* 7, $CH_2CH_3$).

**Reference Example-44(a): (3*S*,3'*R*) and**

**Reference Example 44(b): (3*S*,3'*S*)-3-(3'-Hydroxy-2',2'-dimethyldecanoyl)amino-caprolactam:**

2,2-Dimethyl-3-hydroxy decanoic acid (1.77 mmol) and 1-hydroxybenzotriazole monohydrate (1.77 mmol) were dissolved in THF (10 ml). 1-[3-(Dimethylamino)propyl]-3-ethylcarbodiimide hydrochloride (1.77 mmol) was added and the reaction was stirred at ambient temperature for 4 hours. A solution of (*S,S*)-3-amino-caprolactam hydro-pyrrolidine-5-carboxylate 2 (2 mmol) and $Na_2CO_3$ (6 mmol) in water (15 ml) was added and the reaction was stirred for 18 hours. The reaction solvent was then removed *in vacuo* and the residue was partitioned between water and ethyl acetate. The ethyl acetate layer was washed with pH 2 buffer (0.5 M $Na_2SO_4$ / 0.5 M $NaHSO_4$) and dilute aqueous sodium hydroxide, and then dried over $Na_2SO_4$ and reduced *in vacuo*. The residue was chromatographed on silica gel (25% ethyl acetate in hexanes to 100% ethyl acetate) to give a mixture of (3*S*,3',*R*) and (3*S*,3'*S*)-3-(3'-hydroxy-2',2'-dimethyldecanoyl) amino-caprolactams (557 mg, 88%); $\delta_H$ (500 MHz, $CDCl_3$) 7.28 (1H, d, *J* 6, N*H*CH one isomer), 7.25 (1H, d, *J* 6, NHCH, one isomer), 6.62-6.48 (1H, br m, N*H*$CH_2$, both isomers), 4.53-4.42 (1H, m, NCH, both isomers), 3.77 (1H, br d, *J*, 6, OH, one isomer), 3.63 (1H, br d, *J*, 6, OH, one isomer), 3.47-3.36 (1H, m, C*H*OH, both isomers), 3.32-3.17 (2H, m, $NCH_2$, both isomers), 2.07-1.92 (2H, m, lactam CH ×2, both isomers), 1.87-1.71 (2H, m, lactam CH ×2, both isomers), 1.60-1.17 (21H, m, lactam CH ×2 + chain $(CH_2)_8$ + $CH_3$, both isomers), 1.14 (3H, s, $CCH_3$, both isomers) and 0.84 (3H, t, *J* 7, $CH_2CH_3$, both isomers); $\delta_C$ (125 MHz, $CDCl_3$) 177.6, 177.2,175.8 (CO, both isomers), 77.8. 77.4 (CHOH), 52.1 (NCH, both isomers), 45.9, 45.8 ($C(CH_3)_2$), 42.1, 42.0 ($NCH_2$), 31.9 (×2) 31.6, 31.3, 30.9, 29.6 (×4), 29.3, 28.8, 27.9, 26.7, 26.6, 22.6 ($CH_2$), 23.7, 23.5, 21.1, 20.4 and 14.1 ($CH_3$);

**Reference Example 45: 2,2-Dimethyl-3-(tetrahydropyran-2-yloxy)-propionic acid (Intermediate)**

2,2-Dimethyl-3-hydroxy propionic acid (100 mmol) and 3,4-dihydro-2*H*-pyran (210 mmol) were dissolved in dichloromethane (50 ml), and para-toluenesulfonic acid (10 mg) was added and the reaction was stirred at ambient temperature for 3 hours. The reaction solvent was then removed and the residue was dissolved in methanol (100 ml). A solution of KOH (120 mmol) in water (30 ml) was added and the reaction was heated at reflux for 18 hours. The reaction solvent was removed *in vacuo* and the residue was partitioned between water and diethyl ether. The aqueous layer was acidified with pH 2 buffer (0.5 M $Na_2SO_4$ / 0.5 M $NaHSO_4$) and then extracted with diethyl ether. The diethyl ether layer was then dried over $Na_2SO_4$ and the solvent was removed *in vacuo* to give 2,2-dimethyl-3-(tetrahydropyran-2-yloxy)-propionic acid as an oil (20.0 g, >95%): $\delta_H$ (400 MHz, $CDCl_3$) 4.62 (1H, t, *J* 3.5, $CHO_2$), 3.82 (1H, ddd, *J* 12, 9, 3, ring $CH_2O$), 3.75 (1H, d, *J* 12, chain $CH_2O$), 3.55-3.46 (1H, m, ring $CH_2O$), 3.40 (1H, d, *J* 12, chain $CH_2O$), 1.90-1.45 (6H, m, $(CH_2)_3$), 1.25 (3H, s, $CH_3$) and 1.23 (3H, s, $CH_3$).

**Reference Example 46: (*S*)-(2',2'-Dimethyl-3'-hydroxy-propionyl)-amino-caprolactam**

2,2-Dimethyl-3-(tetrahydropyran-2-yloxy)-propionic acid (4.65 mmol), 1-hydroxybenzotriazole monohydrate

(4.65 mmol) and carbonyl diimidazole (4.50 mmol) were dissolved in THF (30 ml) and the reaction was heated at reflux for 4 hours. After the reaction was cooled to ambient temperature, a solution of (*S,S*)-amino-caprolactam hydro-pyrrolidine-5-carboxylate 2 (5 mmol) and $Na_2CO_3$ (15 mmol) in water (30 ml) was added and the reaction was stirred for 18 hours. The THF was then removed from the reaction by distillation in *vacuo* and the aqueous layer was extracted with ethyl acetate. The ethyl acetate layer was dried over $Na_2SO_4$ and reduced *in vacuo*. The residue was dissolved in MeOH, and acetyl chloride (1 ml) was added. The reaction was stirred at ambient temperature for 18 hours, and then reduced *in vacuo* to give (*S*)-(2'-dimethyl-3'-hydroxy propionyl)amino-caprolactam as a solid (854 mg, 83%); m.p. 97-99 °C; $[\alpha]_D^{25}$ (c = 0.5, $CHCl_3$) +30.0; $\delta_H$ (400 MHz, $CDCl_3$) 7.24 (1H, d, *J* 5.0, CHN*H*), 6.38 (1H, br s, $CH_2N$*H*), 4.49 (1H, dd, *J* 10, 6, C*H*NH), 3.54 (1H, d, *J* 11, C*H*HOH), 3.49 (1H, d, *J* 11, C*H*HOH), 3.33-3.20 (2H, m, $CH_2$NH), 2.03-1.96 (2H, m, 2 × ring CH), 1.87-1.72 (2H, m, 2 × ring CH), 1.50-1.30 (2H, m, 2 × ring CH), 1.20 (3H, s, $CH_3$) and 1.18 (3H, s, $CH_3$); $\delta_C$ (125 MHz, $CDCl_3$) 177.2,176.0 (CO), 69.9 (CHOH), 52.1 (NH*C*HCO), 43.2 (CCO), 41.9 ($CH_2N$), 31.1, 28.8, 27.9 ($CH_2$ lactam), 22.4 and 22.3 ($CH_3$);

**<u>Example 16:</u> (*S*)-(3'-Chloro-2'-(chloromethyl)-2'-methylpropionyl)amino-caprolactam**

(*S,S*)-3-amino-caprolactam hydro-pyrrolidine-5-carboxylate 2 (5 mmol) and $Na_2CO_3$ (15 mmol) in water (15 ml) were added to a solution of 3,3'-dichlompivaloyl chloride (5 mmol) in dichloromethane (15 ml) at ambient temperature and the reaction was stirred for 12 hours. The organic layer was then separated and the aqueous phase was extracted with additional dichloromethane (2 × 25 ml). The combined organic layers were dried over $Na_2SO_4$ and reduced *in vacuo*. The residue was recrystallised from hexane to give (*S*)-(3'-chloro-2'-(chloromethyl)-2'-methylpropionyl)amino-caprolactam (973 mg, 69%); m.p. (hexanes) 95-96 °C; $[\alpha]_D^{25}$ (c = 0.5, $CHCl_3$) +16.4; $\delta_H$ (500 MHz, $CDCl_3$) 7.33 (1H, d, *J* 5.0, CHN*H*), 6.82-6.62 (1H, br m, $CH_2N$*H*), 4.49 (1H, ddd, *J* 11, 5.5, 1.5, C*H*NH), 3.78 (1H, d, *J* 11, C*H*HCl), 3.74 (1H, d, *J* 11, C*H*HCl), 3.69 (1H, d, *J* 11, C*H*HCl), 3.66 (1H, d, *J* 11, C*H*HCl), 3.29-3.17 (2H, m, $CH_2$NH), 2.05 (1H, br s, *J* 13.5, ring CH), 2.01-1.93 (1H, m, ring CH), 1.87-1.71 (2H, m, 2 × ring CH) and 1.49-1.31 (5H, m, 2 × ring CH + $CH_3$); $\delta_C$ (125 MHz, $CDCl_3$) 175.4, 170.6 (CO), 52.6 (NH*C*HCO), 49.1 (*C*CO), 48.7,48.6 ($CH_2Cl$), 42.1 ($CH_2N$), 31.1, 28.8, 27.9 ($CH_2$ lactam) and 18.9 ($CH_3$).

**<u>Pharmacological study of the products of the invention</u>**

<u>Inhibition of MCP-1 induced leukocyte migration</u>

*Assay principle*

The biological activity of the compounds of the current invention may be demonstrated using any of a broad range of functional assays of leukocyte migration in vitro, including but not limited to Boyden chamber and related transwell migration assays, under-agarose migration assays and direct visualisation chambers such as the Dunn Chamber.

For example, to demonstrate the inhibition of leukocyte migration in response to chemokines (but not other chemoattractants) the 96-well format micro transwell assay system from Neuroprobe (Gaithersburg, MD, USA) has been used. In principle, this assay consists of two chambers separated by a porous membrane. The chemoattractant is placed in the lower compartment and the cells are placed in the upper compartment. After incubation for a period at 37°C the cells move towards the chemoattractant, and the number of cells in the lower compartment is proportional to the chemoattractant activity (relative to a series of controls).

This assay can be used with a range of different leukocyte populations. For example, freshly prepared human peripheral blood leukocytes may used. Alternatively, leukocyte subsets may be prepared, including polymorphonuclear cells or lymphocytes or monocytes using methods well known to those skilled in the art such as density gradient centrifugation or magnetic bead separations.

Alternatively, immortal cell lines which have been extensively validated as models of human peripheral blood leukocytes may be used, including, but not limited to THP-1 cells as a model of monocytes or Jurkat cells as model of naive T cells.

Although a range of conditions for the assay are acceptable to demonstrate the inhibition of chemokine-induced leukocyte migration, a specific example is hereby provided.

*Materials*

The transwell migration systems are manufactured by Neuroprobe, Gaithersburg, MD, USA.
The plates used are ChemoTx plates (Neuroprobe 101-8) and 30 μl clear plates (Neuroprobe MP30).
Geys' Balanced Salt Solution is purchased from Sigma (Sigma G-9779).
Fatty acid-free BSA is purchased from Sigma (Sigma A-8806).
MTT, i.e. 3-(4,5-dimethylthiazol-2-yl)-2,5-diphenyltetrazolium bromide, is purchased from Sigma (Sigma M-5655).
RPMI-1640 without phenol red is purchased from Sigma (Sigma R-8755).
The THP-1 cell line (European Cell culture Collection) were used as the leukocyte cell population.

*Test protocol*

The following procedure is used for testing the invention compounds for MCP-1 induced leukocyte migration:

First, the cell suspension to be placed in the upper compartment is prepared. The THP-1 cells are pelleted by centrifugation (770 x g; 4 mins) and washed with Geys Balanced Salt Solution with 1mg/ml BSA (GBSS + BSA). This wash is then repeated, and the cells repelleted before being resuspended in a small volume of GBSS + BSA for counting, for example using a standard haemocytometer.

The volume of GBSS + BSA is then adjusted depending on the number of cells present so that the cells are at final density of 4.45 x $10^6$ cells per ml of GBSS + BSA. This ensures that there are 100,000 THP-1 cells in each 25μl of the solution that will be placed in the upper chamber of the plate.

To test a single compound for its ability to inhibit MCP-1 induced migration, it is necessary to prepare two lots of cells. The suspension of THP-1 cells at 4.45 x $10^6$ cells/ml is divided into two pots. To one pot the inhibitor under test is added at an appropriate final concentration, in an appropriate vehicle (for example at 1μM in not more than 1% DMSO). To the second pot an equal volume of GBSS + BSA plus vehicle as appropriate (e.g. not more than 1% DMSO) is added to act as a control.

Next, the chemoattractant solution to be placed in the lower compartment is prepared. MCP-1 is diluted in GBSS + BSA to give a final concentration of 25 ng/ml. This is divided into two pots, as for the cell suspension. To one pot, the test compound is added to the same final concentration as was added to the cell suspension, while to the other pot an equal volume of GBSS + BSA plus vehicle as appropriate (e,g. not more than 1% DMSO) is added.

Note that the volume of liquid that needs to be added to make the addition of the text compound needs to be taken into account, when establishing the final concentration of MCP-1 in the solution for the lower compartment and the final concentration of cells in the upper compartment.

Once the chemoattractant solutions for the lower wells and cell solutios for the upper chambers have been prepared, the migration chamber should be assembled. Place 29 μl of the appropriate chemoattractant solution into the lower well of the chamber. Assays should be performed with at least triplicate determinations of each condition. Once all the lower chambers have been filled, apply the prous membrane to the chamber in accordance with the manufacturer's instructions. Finally, apply 25 μl of the appropriate cell solution to each upper chamber. A plastic lid is placed over the entire apparatus to prevent evaporation.

The assembled chamber is incubated at 37 °C, 5% $CO_2$, for 2 hours. A suspension of cells in GBSS + BSA is also incubated under identical conditions in a tube: these cells will be used to construct a standard curve for determining the number of cells that have migrated to the lower chamber under each condition.

At the end of the incubation, the liquid cell suspension is gently removed from the upper chamber, and 20μl of ice-cold 20mM EDTA in PBS is added to the upper chamber, and the apparatus is incubated at 4°C for 15 mins. This procedure causes any cells adhering to the underside of the membrane to fall into the lower chamber.

After this incubation the filter is carefully flushed with GBSS + BSA to wash off the EDTA, and then the filter is removed.

The number of cells migrated into the lower chamber under each condition can then be determined by a number of methods, including direct counting, labelling with fluorescent or radioactive markers or through the use of a vital dye. Typically, we utilise the vital dye MTT. 3 μl of stock MTT solution are added to each well, and then the plate is incubated at 37 °C for 1-2 hours during which time dehydrogenase enzymes within the cells convert the soluble MTT to an insoluble blue formazan product that can be quantified spectrophotometrically.

In parallel, an 8-point standard curve is set up. Starting with the number of cells added to each upper chamber (100,000) and going down in 2-fold serial dilutions in GBSS + BSA, the cells are added to a plate in 25 μl, with 3 μl of MTT stock solution added.

The standard curve plate is incubated along side the migration plate.

At the end of this incubation, the liquid is carefully removed from the lower chambers, taking care not to disturb

the precipitated formazan product. After allowing to air dry briefly, 20μl of DMSO is added to each lower chamber to solubilise the blue dye, and absorbance at 595nm is determined using a 96-well plate reader. The absorbance of each well is then interpolated to the standard curve to estimate the number of cells in each lower chamber.

The MCP-1 stimulated migration is determined by subtracting the average number of cells that reached the lower compartment in wells where no MCP-1 was added from the average number of cells that reached the lower compartment where MCP-1 was present at 25ng/ml.

The impact of the test substance is calculated by comparing the MCP-1-induced migration which occurred in the presence or absence of various concentrations of the test substance. Typically, the inhibition of migration is expressed as a percentage of the total MCP-1 induced migration which was blocked by the presence of the compound For most compounds, a dose-response graph is constructed by determining the inhibition of MCP-1 induced migration which occurs at a range of different compound concentrations (typically ranging from InM to 1μM or higher in the case of poorly active compounds). The inhibitory activity of each compound is then expressed as the concentration of compound required to reduce the MCP-1-induced migration by 50% (the $ED_{50}$ concentration).

*Results*

The compounds of examples 1 to 14 and 16, and reference examples 1 to 7, 9 to 20, 25, 26, 27, 30, 34, 35, 40, 41 and 46, were tested and were shown to have an $ED_{50}$ of 100 nM or less in this test.

*Enantioselectivity*

The (S)- and (R)- enantiomers of three different members of the aminocaprolactam series were synthesised to determine whether the biological activity showed enantioselectivity.

The comparison was made between the compounds of reference examples 1 and 7, between the compounds of reference examples 10 and 11, and between the compounds of examples 1 and 2.

The dose-response curves for each of the four compounds of reference examples 1, 7, 10 and 11 as inhibitors of MCP-1 induced THP-1 cell migration were determined using the transwell migration assay and are shown in Figure 1. In both cases, the (S)-enantiomer was significantly (10-50 fold) more active than the (R)-enantiomer.

Very similar data was obtained using the compounds of examples 1 and 2, such that the (S)-enantiomer was significantly (10-50 fold) more active than the (R)-enantiomer.

These data, for three example members of the aminocaprolactam series, demonstrate that for the application of the compounds of the present invention as anti-inflammatory agents in vivo it is preferable to use the pure (S)-enantiomer of the compound, rather than the racemic mixture of the two enantiomere or the pure (R)-enantiomer.

*In vivo* activity of compounds of the invention:

The anti-inflammatory activity of the compounds of the invention was determined in vivo using a sub-lethal LPS-induced endotoxemia model. Adult male CD-1 mice (n=6 per group) were pretreated with various agents (vehicle, compounds of the invention or positive control agents such as the steroid dexamethasone) by sub-cutaneous injection 30 minutes prior to an acute inflammatory challenge with 750μg of bacterial lipopolysaccharide (from *E. Coli* 01111:B4; Sigma catalog # L-4130) via the intraperitoneal route. The vehicle in each case was 0.6% DMSO, 1% carboxymethyl cellulose, or alternatively 1 % carboxymethylcellulose alone. For some of the compounds, this formulation results in a finely divided suspension or slurry rather than a clear solution. Two hours after LPS challenge, the animals were sacrificed and blood was drawn by cardiac puncture. The level of the pro-inflammatory cytokine TNF-alpha was determined using the Quantikine M ELISA (R&D Systems) for murine TNF-alpha, and reported as the mean ± standard error for each group.

Mice which did not receive the LPS challenge had very low circulating levels of TNF-alpha (typically 10 pg/ml). By 2 hours after the LPS challenge, this had increased by more than 1,000 fold to an average of 20,000 pg/ml, representing a sensitive index of inflammatory activation. Pre-treatment with known anti-inflammatory drugs (such as the steroid dexamethasone) reduced the stimulation of TNF-alpha by up to 85-95%, depending on the dose given.

Example compounds 1 and 5, and reference example compounds 7, 9 and 10 were all tested in this model. All five compounds were able to block TNF-alpha stimulation to a similar extent to dexamethosone, when given at a suitable dose. All five compounds were maximally active at a dose below 1mg/kg.

In a separate series of experiments, the compounds of the invention were administered to animals as an oral suspension, formulated in the same way as for the sub-cutaneous dosing experiments, followed 1 hour later with the LPS challenge exactly as described above. Example compounds 1 and 5, and reference example compounds 7, 9 and 10were all tested in this model, and all five compounds were able to block TNF-alpha stimulation when administered via the oral route at a suitable dose. All five compounds were maximally active at a dose below 30mg/kg.

## Claims

1. Use of a compound of general formula (I) or a pharmaceutically acceptable salt thereof in the preparation of a medicament for the treatment of an inflammatory disorder selected from the group consisting of multiple sclerosis, stroke, coronary artery disease, myocardial infarction, unstable angina pectoris, atherosclerosis, vasculitis, viral infection or replication, asthma, osteoporosis, tumor growth, rheumatoid arthritis, organ transplant rejection, delayed graft or organ function, psoriasis, skin wounds, malaria, tuberculosis, allergies, Alzheimer's disease, immune response suppression, ALS, and fibrosis:

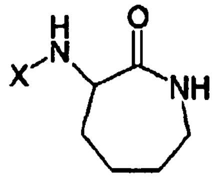

**(I)**

wherein
X is $-CO-R^1$ or $-SO_2-R^2$,
$R^1$ is an alkyl, haloalkyl, alkenyl or alkynyl radical of 4 to 20 carbon atoms which is di-substituted at the carbon of $R^1$ attached directly to the carbonyl carbon of X with the same or different groups selected from: alkyl, haloalkyl, alkoxy, haloalkoxy, alkenyl, alkynyl or alkylamino radicals, such that $R^1$ is 4 to 20 carbon atoms; and
$R^2$ is a linear or branched alkyl radical of 4 to 20 carbon atoms.

2. The use according to claim 1, wherein the compound is a compound of formula **(I')** or a pharmaceutically acceptable salt thereof:

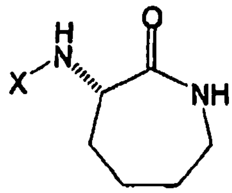

**(I')**

wherein X has the same meaning as in claim 1.

3. A pharmaceutical composition comprising, as active ingredient, a compound of formula (I) or a pharmaceutically acceptable salt thereof, and at least one pharmaceutically acceptable excipient and/or carrier:

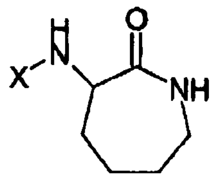

**(I)**

wherein
X is $-CO-R^1$ or -SO2-$R^2$,
$R^1$ is an alkyl, haloalkyl, alkenyl or alkynyl radical of 4 to 20 carbon atoms which is di-substituted at the carbon of $R^1$ attached directly to the carbonyl carbon of X with the same or different groups selected from: alkyl, haloalkyl,

alkoxy, haloalkoxy, alkenyl, alkynyl or alkylamino radicals, such that $R^1$ is 4 to 20 carbon atoms; and
$R^2$ is a linear or branched alkyl radical of 4 to 20 carbon atoms.

**4.** The pharmaceutical composition according to claim 3, wherein the compounds is a compound of formula (I') or a pharmaceutically acceptable salt thereof

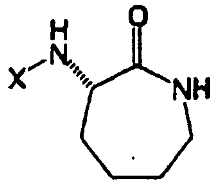

(I')

wherein X has the same meaning as in claim 3.

**5.** A compound of general formula (I):

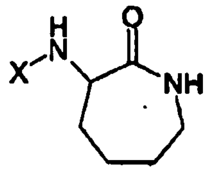

(I)

wherein
X is -CO-$R^1$ or -$SO_2$-$R^2$,
$R^1$ is an alkyl, haloalkyl, alkenyl or alkynyl radical of 4 to 20 carbon atoms which is di-substituted at the carbon of $R^1$ attached directly to the carbonyl carbon of X with the same or different groups selected from: alkyl, haloalkyl, alkoxy, haloalkoxy, alkenyl, alkynyl or alkylamino radicals, such that $R^1$ is 4 to 20 carbon atoms; and
**$R^2$** is a linear or branched alkyl radical of 4 to 20 carbon atoms.

**6.** The compound according to claim 5, having a general formula (I'):

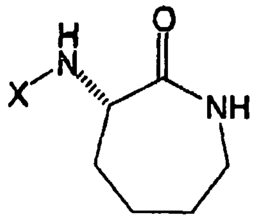

(I')

wherein X has the same meaning as in claim 5.

**7.** The use according to claim 1, or pharmaceutical composition according to claim 3, or compound according to claim 5, wherein the compound is (S)-3-(2',2'-dimethyl-dodecanoyl)amino-caprolactam or a pharmaceutically acceptable salt thereof.

**8.** The use according to claim 1, or pharmaceutical composition according to claim 3, or compound according to claim 5, wherein the compound is selected from the group consisting of:

- (*R*)-3-(2',2'-Dimethyl-dodecanoyl)amino-caprolactam;

- (*S*)-3-(2',2' -Dimethyl-pentanoyl)amino-caprolactam;
- (*S*)-3-(2',2'-Dimethyl-pent-4-enoyl)amino-caprolactam;
- (*S*)-3-(2',2'-Dimethyl-propionyl)amino-caprolactem;
- (*S*)-3-(2',2'-Dimethyl-butyryl)amino-caprolactam;
- (*S*,E)-3-(2',2'-Dimethyl-dodec-4'-enoyl)amino-caprolactam;
- (*S*)-3-(2',2',5'-Trimethyl-hex-4'-enoyl)amino-caprolactam;
- (*S*)-3-(2',2',5'-Trimethyl-hexanoyl)amino-caprolactam;
- (*S*)-3-(Decanesulfonyl)amino-caprolactam;
- (*S*)-3-(Dodecanesulfonyl)amino-caprolactatn;
- (*S*)-3-(Tetradecanesulfonyl)amino-caprolactam;
- (*S*)-3-(Hexadecanesulfonyl)amino-caprolactam;
- (*S*)-3-(Octadecanesulfonyl)amino-caprolactam;
- (*S*)-(3'-Chloro-2'-(chloromethyl)-2'-methylpropionyl)amino-caprolactam;

and pharmaceutically acceptable salts thereof.

**9.** The use according to claim 1, or pharmaceutical composition according to claim 3, or compound according to claim 5, wherein the compound is selected from the group consisting of: (*S*)-3-(2',2'-dimethyl-dodecanoyl)amino-caprolactam, (S')-3-(2',2'-dimethyl-piopionyl)amino-caprolactam and pharmaceutically acceptable salts thereof.

**10.** Compounds, compositions and uses according to any of claims 1-9, wherein the 4 to 20 carbon atoms comprise 5 to 20 carbon atoms, 8 to 20 carbon atoms, 9 to 20 carbon atoms, 10 to 18 carbon atoms, 12 to 18 carbon atom, 13 to 18 carbon atoms, 14 to 18 carbon atoms, or 13 to 17 carbon atoms.

**11.** A compound as defined in any of claims 5-10 for use in the treatment of an inflammatory disorder selected from the group consisting of multiple sclerosis, stroke, coronary artery disease, myocardial infarction, unstable angina pectoris, atherosclerosis, vasculitis, viral infection or replication, asthma, osteoporosis, tumor growth, rheumatoid arthritis, organ transplant rejection, delayed graft or organ function, psoriasis, skin wounds, malaria, tuberculosis, allergies, Alzheimer's disease, immune response suppression,ALS, and fibrosis.

**Patentansprüche**

**1.** Verwendung einer Verbindung der allgemeinen Formel (I) oder eines pharmazeutisch akzeptablen Salzes davon bei der Herstellung eines Medikaments zur Behandlung einer Entzündungsstörung, die ausgewählt ist aus der Gruppe bestehend aus Multipler Sklerose, Schlaganfall, koronarer Arterienkrankheit, Herzinfarkt, instabiler Angina pectoris, Atherosklerose, Vaskulitis, Virusinfektion oder - replikation, Asthma, Osteoporose, Tumorwachstum, Rheumatoidarthritis, Organtransplantatabstoßung, verzögerter Transplantat- oder Organfunktion, Psoriasis, Hautwunden, Malaria, Tuberkulose, Allergien, Alzheimer Krankheit, Immunreaktionsunterdrückung, ALS und Fibrose:

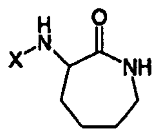

(I)

wobei
X -CO-$R^1$ oder -$SO_2$-$R^2$ ist,
$R^1$ ein Alkyl-, Haloalkyl-, Alkenyl- oder Alkynylradikal mit 4 bis 20 Kohlenstoffatomen ist, das disubstituiert ist an dem Kohlenstoff von $R^1$, der direkt am Carbonylkohlenstoff von X angefügt ist, durch die gleichen oder unterschiedliche Gruppen, die ausgewählt sind aus: Alkyl-, Haloalkyl-, Alkoxy-, Haloalkoxy-, Alkenyl-, Alkynyl- oder Alkylaminoradikalen, so dass $R^1$ 4 bis 20 Kohlenstoffatome umfasst; und
$R^2$ ein lineares oder verzweigtes Alkylradikal mit 4 bis 20 Kohlenstoffatomen ist.

**2.** Verwendung nach Anspruch 1, wobei die Verbindung eine Verbindung der Formel (I') oder ein pharmazeutisch akzeptables Salz davon ist:

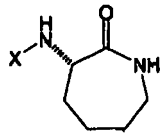

(I')

wobei X die gleiche Bedeutung wie in Anspruch 1 hat.

**3.** Pharmazeutische Zusammensetzung, die als Wirkstoff eine Verbindung der Formel (I) oder ein pharmazeutisch akzeptables Salz davon und wenigstens einen pharmazeutisch akzeptablen Exzipienten und/oder Träger umfasst:

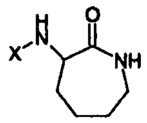

(I)

wobei
X -CO-$R^1$ oder -$SO_2$-$R^2$ ist,
$R^1$ ein Alkyl-, Haloalkyl-, Alkenyl- oder Alkynylradikal mit 4 bis 20 Kohlenstoffatomen ist, das disubstituiert ist an dem Kohlenstoff von $R^1$, der direkt am Carbonylkohlenstoff von X angefügt ist, durch die gleichen oder unterschiedliche Gruppen, die ausgewählt sind aus: Alkyl-, Haloalkyl-, Alkoxy-, Haloalkoxy-, Alkenyl-, Alkynyl- oder Alkylaminoradikalen, so dass $R^1$ 4 bis 20 Kohlenstoffatome umfasst; und
$R^2$ ein lineares oder verzweigtes Alkylradikal mit 4 bis 20 Kohlenstoffatomen ist.

**4.** Pharmazeutische Zusammensetzung nach Anspruch 3, wobei die Verbindung eine Verbindung der Formel (I') oder ein pharmazeutisch akzeptables Salz davon ist:

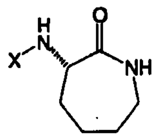

(I')

wobei X die gleiche Bedeutung wie in Anspruch 3 hat.

**5.** Verbindung der allgemeinen Formel (I):

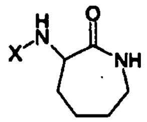

(I)

wobei
X -CO-$R^1$ oder -$SO_2$-$R^2$ ist,
$R^1$ ein Alkyl-, Haloalkyl- oder Alkynylradikal mit 4 bis 20 Kohlenstoffatomen ist, das disubstituiert ist an dem Kohlenstoff von $R^1$, der direkt am Carbonylkohlenstoff von X angefügt ist, durch die gleichen oder unterschiedliche Gruppen, die ausgewählt sind aus: Alkyl-, Haloalkyl-, Alkoxy-, Haloalkoxy-, Alkenyl-, Alkynyl- oder Alkylaminoradikalen, so dass $R^1$ 4 bis 20 Kohlenstoffatome umfasst; und
$R^2$ ein lineares oder verzweigtes Alkylradikal mit 4 bis 20 Kohlenstoffatomen ist.

**6.** Verbindung nach Anspruch 5 mit der Formel (I'):

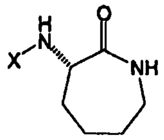

(I')

wobei X die gleiche Bedeutung wie in Anspruch 5 hat.

7. Verwendung nach Anspruch 1 oder pharmazeutische Zusammensetzung nach Anspruch 3 oder Verbindung nach Anspruch 5, wobei die Verbindung (S)-3-(2',2'-Dimethyl-dodecanoyl)amino-caprolactam oder ein pharmazeutisch akzeptables Salz davon ist.

8. Verwendung nach Anspruch 1 oder pharmazeutische Zusammensetzung nach Anspruch 3 oder Verbindung nach Anspruch 5, wobei die Verbindung ausgewählt ist aus der Gruppe bestehend aus:

- (*R*)-3-(2',2'-Dimethyl-dodecanoyl)amino-caprolactam;
- (*S*)-3-(2',2'-Dimethyl-pentanoyl)amino-caprolactam;
- (*S*)-3-(2',2'-Dimethyl-pent-4-enoyl)amino-caprolactam;
- (*S*)-3-(2',2'-Dimethyl-propionyl)amino-caprolactam;
- (*S*)-3-(2',2'-Dimethyl-butyryl)amino-caprolactam;
- (*S,E*)-3-(2',2'-Dimethyl-dodec-4'-enoyl)amino-caprolactam;
- (*S*)-3-(2',2',5'-Trimethyl-hex-4'-enoyl)amino-caprolactam;
- (*S*)-3-(2',2',5'-Trimethyl-hexanoyl)amino-caprolactam;
- (*S*)-3-(Decansulfonyl)amino-caprolactam;
- (*S*)-3-(Dodecansulfonyl)amino-caprolactam;
- (*S*)-3-(Tetradecansulfonyl)amino-caprolactam;
- (*S*)-3-(Hexadecansulfonyl)amino-caprolactam;
- (*S*)-3-(Octadecansulfonyl)amino-caprolactam;
- (*S*)-(3'-Chlor-2'-(chlormethyl)-2'-methylpropionyl)amino-caprolactam;

und pharmazeutisch akzeptablen Salzen davon.

9. Verwendung nach Anspruch 1 oder pharmazeutische Zusammensetzung nach Anspruch 3 oder Verbindung nach Anspruch 5, wobei die Verbindung ausgewählt ist aus der Gruppe bestehend aus: (*S*)-3-(2',2'-Dimethyl-dodecanoyl) amino-caprolactam, (*S*)-3-(2',2'-Dimethyl-propionyl)amino-caprolactam und pharmazeutisch akzeptablen Salzen davon.

10. Verbindungen, Zusammensetzungen und Verwendungen nach einem der Ansprüche 1-9, wobei die 4 bis 20 Kohlenstoffatome 5 bis 20 Kohlenstoffatome, 8 bis 20 Kohlenstoffatome, 9 bis 20 Kohlenstoffatome, 10 bis 18 Kohlenstoffatome, 12 bis 18 Kohlenstoffatome, 13 bis 18 Kohlenstoffatome, 14 bis 18 Kohlenstoffatome oder 13 bis 17 Kohlenstoffatome umfassen.

11. Verbindung nach einem der Ansprüche 5-10 zur Verwendung bei der Behandlung einer Entzündungsstörung, die ausgewählt ist aus der Gruppe bestehend aus Multipler Sklerose, Schlaganfall, koronarer Arterienkrankheit, Herzinfarkt, instabiler Angina pectoris, Atherosklerose, Vaskulitis, Virusinfektion oder -replikation, Asthma, Osteoporose, Tumorwachstum, Rheumatoidarthritis, Organtransplantatabstoßung, verzögerter Transplantat- oder Organfunktion, Psoriasis, Hautwunden, Malaria, Tuberkulose, Allergien, Alzheimer Krankheit, Immunreaktionsunterdrückung, ALS und Fibrose.

**Revendications**

1. Utilisation d'un composé de formule générale (I) ou d'un sel pharmacologiquement acceptable de celui-ci dans la préparation d'un médicament pour le traitement d'une affection inflammatoire sélectionnée parmi le groupe constitué par la sclérose en plaques, l'accident vasculaire cérébral, la coronaropathie, l'infarctus du myocarde, l'angor instable, l'athérosclérose, la vascularite, l'infection ou la réplication virale, l'asthme, l'ostéoporose, la croissance tumorale, la polyarthrite rhumatoïde, le rejet de greffe d'organe, la fonction retardée d'une greffe ou d'un organe, le psoriasis,

les plaies cutanées, le paludisme, la tuberculose, les allergies, la maladie d'Alzheimer, la suppression de la réponse immunitaire, la SLA et la fibrose :

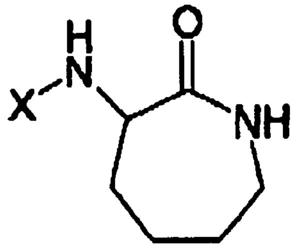

(I)

dans laquelle
X est -CO-$R^1$ ou -$SO_2$-$R^2$,
$R^1$ est un radical alkyle, haloalkyle, alkényle ou alkynyle renfermant 4 à 20 atomes de carbone qui est disubstitué au niveau du carbone de $R^1$ directement fixé au carbone carbonyle de X avec des groupes identiques ou différents sélectionnés parmi le groupe constitué par : les radicaux alkyle, haloalkyle, alkoxy, haloalkoxy, alkényle, alkynyle ou alkylamino, de telle sorte que $R^1$ renferme 4 à 20 atomes de carbone ; et
$R^2$ est un radical alkyle linéaire ou ramifié renfermant 4 à 20 atomes de carbone.

**2.** Utilisation selon la revendication 1, dans laquelle le composé est un composé de formule (I') ou un sel pharmaceutiquement acceptable de celui-ci :

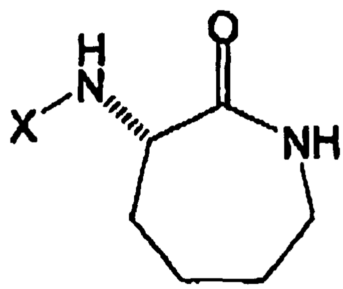

**(I')**

dans laquelle X a la même signification qu'à la revendication 1.

**3.** Composition pharmaceutique comprenant, à titre de constituant actif, un composé de formule **(I)** ou un sel pharmaceutiquement acceptable de celui-ci, et au moins un excipient et/ou un vecteur pharmaceutiquement acceptable :

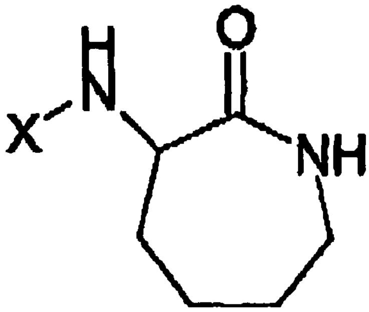

(I)

dans laquelle
X est -CO-$R^1$ ou -$SO_2$-$R^2$,
$R^1$ est un radical alkyle, haloalkyle, alkényle ou alkynyle renfermant 4 à 20 atomes de carbone qui est disubstitué au niveau du carbone de $R^1$ directement fixé au carbone carbonyle de X avec des groupes identiques ou différents sélectionnés parmi le groupe constitué par : les radicaux alkyle, haloalkyle, alkoxy, haloalkoxy, alkényle, alkynyle ou alkylamino, de telle sorte que $R^1$ renferme 4 à 20 atomes de carbone ; et
$R^2$ est un radical alkyle linéaire ou ramifié renfermant 4 à 20 atomes de carbone.

4. Composition pharmaceutique selon la revendication 3, dans laquelle le composé est un composé de formule (I') ou un sel pharmaceutiquement acceptable de celui-ci:

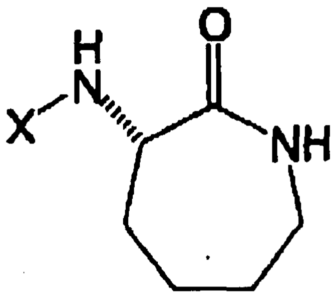

(I')

dans laquelle X a la même signification qu'à la revendication 3.

5. Composé de formule générale (I) :

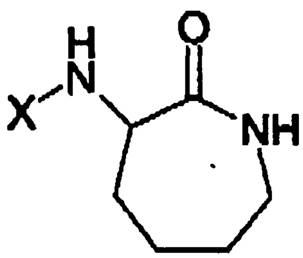

(I)

dans laquelle
X est -CO-$R^1$ ou -$SO_2$-$R^2$,
$R^1$ est un radical alkyle, haloalkyle, alkényle ou alkynyle renfermant 4 à 20 atomes de carbone qui est disubstitué au niveau du carbone de $R^1$ directement fixé au carbone carbonyle de X avec des groupes identiques ou différents sélectionnés parmi le groupe constitué par : les radicaux alkyle, haloalkyle, alkoxy, haloalkoxy, alkényle, alkynyle ou alkylamino, de telle sorte que $R^1$ renferme 4 à 20 atomes de carbone ; et
$R^2$ est un radical alkyle linéaire ou ramifié renfermant 4 à 20 atomes de carbone.

**6.** Composé selon la revendication 5, ayant une formule générale (I') :

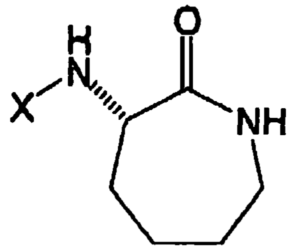

**(I')**

dans laquelle X a la même signification qu'à la revendication 5.

**7.** Utilisation selon la revendication 1, ou composition pharmaceutique selon la revendication 3, ou composé selon la revendication 5, dans laquelle le composé est le (S)-3-(2',2'-diméthyl-dodécanoyl)amino-caprolactame ou un sel pharmaceutiquement acceptable de celui-ci.

**8.** Utilisation selon la revendication 1, ou composition pharmaceutique selon la revendication 3, ou composé selon la revendication 5, dans laquelle le composé est sélectionné parmi le groupe constitué par :

- (R)-3-(2',2'-diméthyl-dodécanoyl)amino-caprolactame ;
- (S)-3-(2',2'-diméthyl-pentanoyl)amino-caprolactame ;
- (S)-3-(2',2'-diméthyl-pent-4-énoyl)amino-caprolactame ;
- (S)-3-(2',2'-diméthyl-propionyl)amino-caprolactame ;
- (S)-3-(2',2'-diméthyl-butyryl)amino-caprolactame ;
- (S,E)-3-(2',2'-diméthyl-dodec-4'-énoyl)amino-caprolactame ;
- (S)-3-(2',2', 5'-triméthyl-hex-4'-énoyl)amino-caprolactame ;
- (*S*)-3-(2',2',5'-triméthyl-hexanoyl)amino-caprolactame ;
- (*S*)-3-(décanesulfonyl)amino-caprolactame ;
- (*S*)-3-(dodécanesulfonyl)amino-caprolactame ;
- (*S*)-3-(tétradécanesulfonyl)amino-caprolactame ;
- (*S*)-3-(hexadécanesulfonyl)amino-caprolactame ;
- (*S*)-3-(octadécanesulfonyl)amino-caprolactame ;
- (*S*)-(3'-chloro-2'-(chlorométhyl)-2'-méthylpropionyl)amino-caprolactame ;

et des sels pharmaceutiquement acceptables de ceux-ci.

**9.** Utilisation selon la revendication 1, ou composition pharmaceutique selon la revendication 3, ou composé selon la revendication 5, dans laquelle le composé est sélectionné parmi le groupe constitué par: (S)-3-(2',2'-dimétyl-dodécanoyl)amino-caprolactame, (*S*)-3-(2',2'-diméthyl-propionyl)amino-caprolactame et des sels pharmaceutiquement acceptables de ceux-ci.

**10.** Composés, compositions et utilisations selon l'une quelconque des revendications 1 à 9, dans lesquels les 4 à 20 atomes de carbone renferment 5 à 20 atomes de carbone, 8 à 20 atomes de carbone, 9 à 20 atomes de carbone, 10 à 18 atomes de carbone, 13 à 18 atomes de carbone, 14 à 18 atomes de carbone, ou 13 à 17 atomes de carbone.

**11.** Composé tel que défini dans l'une quelconque des revendications 5 à 10, à utiliser dans le traitement d'une affection inflammatoire sélectionnée parmi le groupe constitué par la sclérose en plaques, l'accident vasculaire cérébral, la coronaropathie, l'infarctus du myocarde, l'angor instable, l'athérosclérose, la vascularite, l'infection ou la réplication virale, l'asthme, l'ostéoporose, la croissance tumorale, la polyarthrite rhumatoïde, le rejet de greffe d'organe, la fonction retardée d'une greffe ou d'un organe, le psoriasis, les plaies cutanées, le paludisme, la tuberculose, les allergies, la maladie d'Alzheimer, la suppression de la réponse immunitaire, la SLA et la fibrose.

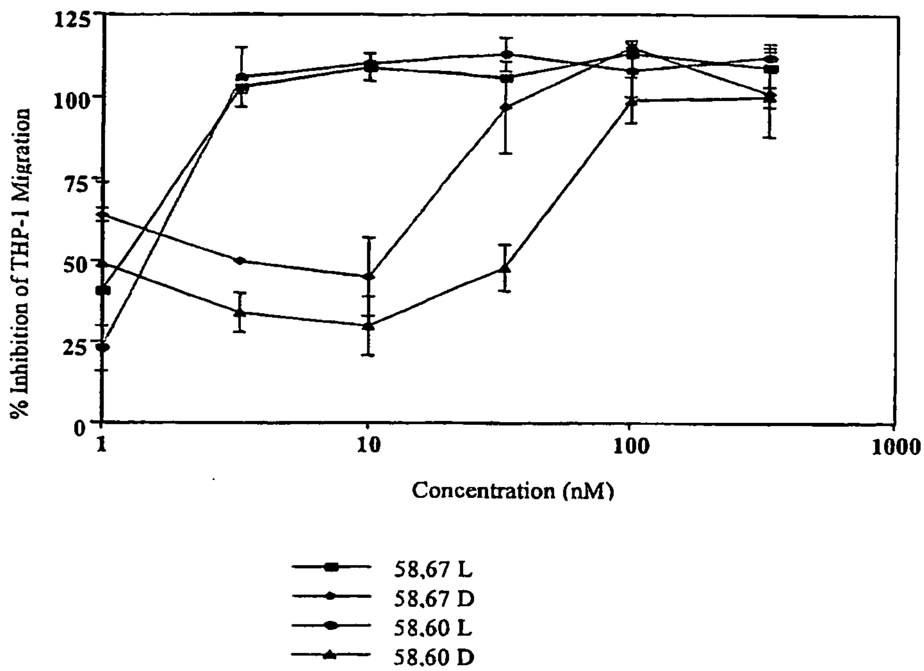
125
100
75
50
25
0
1
10
100
1000
% Inhibition of THP-1 Migration
Concentration (nM)
58,67 L
58,67 D
58,60 L
58,60 D

**FIGURE 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9912968 A
- WO 0042071 A
- JP 09087331 B
- US 6395282 B
- GB 0327775 A
- GB 0417436 A


### Non-patent literature cited in the description


- **Reckless et al.** *Biochem J.,* 1999, vol. 340, 803-811
- **Grainger et al.** *Biochem. Pharm.,* 2003, vol. 65, 1027-1034
- **Fox et al.** *J Med Chem,* 2002, vol. 45, 360-370
- **Weiss et al.** *Research Communications in Psychology, Psychiatry and Behavior,* 1992, vol. 17 (3-4), 153-159
- Salt selection for basic drugs. *Int. J. Pharm.,* 1986, vol. 33, 201-217
- **Boyle et al.** *J. Org. Chem,* 1979, vol. 44, 4841-4847
- **Rezler et al.** *J. Med. Chem,* 1997, vol. 40, 3508-3515
- **Crimmins, M.T ; She, J.** *Synlett,* 2004, 1371-1374
- **Evans et al.** *Tetrahedron Lett.,* 1987, vol. 28, 1123